# EUROPEAN PATENT APPLICATION

(11) **EP 4 628 498 A2**
(43) Date of publication of application: **08.10.2025**
(21) Application number: 25181493.5
(22) Date of filing: 06.11.2020
(51) Int. Cl.: C07K 1/18

(54) **ENGINEERING CHARGE PAIR MUTATIONS FOR PAIRING OF HETERO-IGG MOLECULES**

(30) Priority: 08.11.2019 US 201962933205 P
(62) Divisional of application: 20820597.1
(71) Applicant: Amgen Inc., Thousand Oaks, CA 91320 (US)
(72) Inventor: ESTES, Bram, Thousand Oaks, California 91320-1799 (US); GARCES, Fernando, Thousand Oaks, California 91320-1799 (US); WANG, Zhulun, Thousand Oaks, California 91320-1799 (US); DARIS, Mark, Thousand Oaks, California 91320-1799 (US)
(74) Representative: Lau, Sarah Jane

(57) **Abstract**

The present invention relates to heterodimers comprising antibody CH3 domains and mutations useful for the facilitation of the formation of heterodimers. Methods of optimizing purification of the heterodimers at certain pHs in also disclosed.

## Description

### FIELD OF THE INVENTION

The present invention relates to the field of protein engineering. Specifically, the invention relates to the engineering of charged residues in the crystallizable fragment (Fc) of hetero-IgG molecules to repel homo-Fc formation and drive hetero-Fc formation.

The instant application contains an ASCII "txt" compliant sequence listing which serves as both the computer readable form (CRF) and the paper copy required by 37 C.F.R. Section 1.821(c) and 1.821(e), and is hereby incorporated by reference in its entirety. The name of the "txt" file created on November 6, 2020, is: A-2389-WO-PCT_SEQ_LIST_11062020_ST25, and is 23.6 kb in size.

### BACKGROUND OF THE INVENTION

The natural formation of Fc regions of IgG molecules involves the assembly of two matching Fc chains, independent of the sequences in the Fab arms. Bispecific antibodies have target specificity to two or more different antigens. This is due to the two Fab arms consisting of different sequences. As a result, bispecific molecules with a natural Fc region are prone to assemble heavy chain molecules into three main species, consisting of a monoclonal antibody that binds to one antigen, a monoclonal antibody that binds to a second antigen, and a bispecific antibody that binds to both antigens.

In order to prevent the formation of monoclonal antibodies when these recombinant proteins are being expressed, IgG molecules have been engineered primarily in the CH3 region. Earlier work on driving chain pairing between IgG heavy chains began with the use of the knob in hole strategy in which the contours of the surface area between chains are modified with mutations to larger and smaller residues. A more recent approach that has been implemented broadly is the use of charge pair mutations, in which naturally charged residues at one of the two rotationally symmetrical regions are reversed by mutating the positively charged residue to a negatively charged residue and vice versa. When this approach is applied to one pair of charged residues, it results in two points of repulsion between matching Fc regions and two points of attraction that form salt bridges between differing Fc regions. In our studies, we screened and optimized residues at three pairs of charged residues in the Fc region and also tested neighboring residues for the added benefit of strengthening the drive toward pairing specificity and purification at lower pH.

### SUMMARY OF THE INVENTION

The present invention is directed to a an isolated heteromultimer comprising a heterodimeric CH3 domain comprising a first CH3 domain polypeptide and a second CH3 domain polypeptide, the first CH3 domain polypeptide comprising an amino acid modification at position K360, wherein (i) the first CH3 domain polypeptide further comprises an amino acid modification at position K370, and (ii) the second CH3 domain polypeptide comprises an amino acid modification at position E357, wherein the numbering of amino acid residues is according to the EU index as set forth in Kabat.

In another aspect the present invention is directed to a method of stabilizing an isolated heteromultimer at about pH 5.0 wherein the heteromultimer comprises a heterodimeric CH3 domain comprising a first CH3 domain polypeptide and a second CH3 domain polypeptide, wherein: (i) the first CH3 domain polypeptide comprises an amino acid modification at position K370, and (ii) the second CH3 domain polypeptide comprises an amino acid modification at position E357, the method comprising introducing an amino acid modification at position K360 of the fist CH3 domain, wherein the modification is substitution of K360 for a glutamic or aspartic acid; wherein the numbering of amino acid residues is according to the EU index as set forth in Kabat.

In one embodiment the amino acid modification at position K360 is selected from the group consisting of K360E and K360D.

In one embodiment amino acid modification at position K370 is selected from the group consisting of K370E and K370D.

In one embodiment the amino acid modification at position E357 is selected from the group consisting of E357K, E357H, and E357R.

In one embodiment the amino acid modification at position K360 is K360E, the amino acid modification at position K370 is K370D, and the amino acid modification at position E357 is E357K.

In one embodiment one CH3 domain polypeptide further comprises an amino acid modification at position K409, and the other CH3 domain polypeptide further comprises an amino acid modification at position D399.

In one embodiment the first CH3 domain polypeptide comprises the amino acid modification at position K409, and the second CH3 domain polypeptide comprises the amino acid modification at position D399.

In one embodiment the first CH3 domain polypeptide comprises the amino acid modification at position D399, and the second CH3 domain polypeptide comprises the amino acid modification at position K409.

In one embodiment the amino acid modification at position K409 is selected from the group consisting of K409E and K409D, and wherein the amino acid modification at position D399 is selected from the group consisting of D399K, D399H, and D399R.

In one embodiment the amino acid modification at position K409 is K409D, and wherein the amino acid modification at position D399 is D399K.

In one embodiment the CH3 domain polypeptide which comprises the amino acid modification at position K409, further comprises an amino acid modification at position K392.

In one embodiment the amino acid modification at position K392 is selected from the group consisting of K392E and K392D.

In one embodiment one CH3 domain polypeptide further comprises an amino acid modification at position K439, and the other CH3 domain polypeptide further comprises an amino acid modification at position E356.

In one embodiment the first CH3 domain polypeptide comprises the amino acid modification at position K439, and the second CH3 domain polypeptide comprises the amino acid modification at position E356.

In one embodiment the first CH3 domain polypeptide comprises the amino acid modification at position E356, and the second CH3 domain polypeptide comprises the amino acid modification at position K439.

In one embodiment the amino acid modification at position K439 is selected from the group consisting of K439E and K439D, and wherein the amino acid modification at position E356 is selected from the group consisting of E356K, E356H, and E356R.

In one embodiment the amino acid modification at position K439 is K439E, and wherein the amino acid modification at position E356 is E356K.

In one embodiment the first CH3 domain polypeptide comprises K360E, K370D, K409, and K392D mutations and second CH3 domain polypeptide comprises E357K and D399K mutations.

In one embodiment the first CH3 domain polypeptide further comprises a K439E mutation, and the second CH3 domain polypeptide further comprises a E356K mutation.

In one embodiment the heterodimeric CH3 domain is comprised by an Fc region based on an IgG Fc region.

In one embodiment the IgG Fc region is an IgG1 Fc region.

In one embodiment the heteromultimer is a bispecific or multispecific antibody.

### BRIEF DESCRIPTION OF THE DRAWINGS

Figure 1 depicts a single chain variable region (scFv) fused to an Fc and an Fc chain lacking a warhead. Molecules assembled into homodimers with two scFv warheads (A) or two lacking warheads (C) result in molecular weights of approximately 100 kDa or 50 kDa, respectively. When assembled with the intended hetero-Fc format (B), the molecular weight is approximately 75 kilo Daltons (kDa).
Figure 2 depicts the lead scFv-Fc molecules from screening.
Figure 3 depicts a modified monoclonal antibody. Modifications include the addition of charge pair mutations in the CH3 region and the addition of a DEVD cleavage site in the hinge between one of the Fab and Fc regions.
Figure 4 depicts cation exchange of heterodimers at pH 6.0, 5.6, and 5.0.
Figure 5 depicts cation exchange of heterodimers at pH 6.0, 5.6, and 5.0 compared to K360 mutants.

### DETAILED DESCRIPTION

A "multimeric protein" as used herein refers to a protein containing more than one separate polypeptide or protein chains associated with each other to form a single protein *in vitro* or *in vivo.* The multimeric protein may consist of more than one polypeptide of the same kind to form a "homomultimer." Alternatively, the multimeric protein may also be composed of more than one polypeptide of distinct sequences to form a "heteromultimer." Thus, a "heteromultimer" is a molecule comprising at least a first polypeptide and a second polypeptide, wherein the second polypeptide differs in amino acid sequence from the first polypeptide by at least one amino acid residue. The heteromultimer can comprise a "heterodimer" formed by the first and second polypeptide or can form higher order tertiary structures where more than two polypeptides are present.

As used herein, the term "antigen binding protein" refers to a protein that specifically binds to one or more target antigens. An antigen binding protein can include an antibody and functional fragments thereof. A "functional antibody fragment" is a portion of an antibody that lacks at least some of the amino acids present in a full-length heavy chain and/or light chain, but which is still capable of specifically binding to an antigen. A functional antibody fragment includes, but is not limited to, a Fab fragment, a Fab' fragment, a F(ab')₂ fragment, a Fv fragment, a Fd fragment, and a complementarity determining region (CDR) fragment, and can be derived from any mammalian source, such as human, mouse, rat, rabbit, or camelid. Functional antibody fragments may compete for binding of a target antigen with an intact antibody and the fragments may be produced by the modification of intact antibodies (e.g. enzymatic or chemical cleavage) or synthesized *de novo* using recombinant DNA technologies or peptide synthesis.

"Heavy" and "light" chains refer to the two polypeptides which comprise an IgG. A heavy chain can be broken down into the following domains from N-terminus to C-terminus: VH, CH1, CH2, and CH3. A light chain can be broken down into the following domains from N-terminus to C-terminus: VL and CL. The CH1 and CL domains will interact such that the VH and VL domains form a functional conformation.

An antigen binding protein can also include a protein comprising one or more functional antibody fragments incorporated into a single polypeptide chain or into multiple polypeptide chains. For instance, antigen binding proteins can include, but are not limited to, a diabody (*see, e.g.,* EP 404,097; WO 93/11161; and Hollinger et al., Proc. Natl. Acad. Sci. USA, Vol. 90:6444-6448, 1993); an intrabody; a domain antibody (single VL or VH domain or two or more VH domains joined by a peptide linker; *see* Ward et al., Nature, Vol. 341:544-546, 1989); a maxibody (2 scFvs fused to Fc region, *see* Fredericks et al., Protein Engineering, Design & Selection, Vol. 17:95-106, 2004 and Powers et al., Journal of Immunological Methods, Vol. 251:123-135, 2001); a triabody; a tetrabody; a minibody (scFv fused to CH3 domain; *see* Olafsen et al., Protein Eng Des Sel. , Vol.17:315-23, 2004); a peptibody (one or more peptides attached to an Fc region, *see* WO 00/24782); a linear antibody (a pair of tandem Fd segments (VH-CH1-VH-CH1 ) which, together with complementary light chain polypeptides, form a pair of antigen binding regions, *see* Zapata et al., Protein Eng., Vol. 8:1057-1062, 1995); a small modular immunopharmaceutical (*see* U.S. Patent Publication No. 20030133939); and immunoglobulin fusion proteins (e.g. IgG-scFv, IgG-Fab, 2scFv-IgG, 4scFv-IgG, VH-IgG, IgG-VH, and Fab-scFv-Fc).

In certain aspects, the antigen binding proteins of the present invention are "bispecific" meaning that they are capable of specifically binding to two different antigens. In another aspect, the antigen binding proteins of the present invention are "trispecific" meaning that they are capable of specifically binding to three different antigens. In another aspect, the antigen binding proteins of the present invention are "tetraspecific" meaning that they are capable of specifically binding to four different antigens. As used herein, an antigen binding protein "specifically binds" to a target antigen when it has a significantly higher binding affinity for, and consequently is capable of distinguishing, that antigen, compared to its affinity for other unrelated proteins, under similar binding assay conditions. Antigen binding proteins that specifically bind an antigen may have an equilibrium dissociation constant (K_{D}) ≤ 1 x 10⁻⁶ M. The antigen binding protein specifically binds antigen with "high affinity" when the K_{D} is ≤ 1 x 10⁻⁸ M. In one embodiment, the antigen binding proteins of the invention bind to target antigen(s) with a K_{D} of ≤ 5 x 10⁻⁷ M. In another embodiment, the antigen binding proteins of the invention bind to target antigen(s) with a K_{D} of ≤ 1 x 10⁻⁷ M.

Affinity is determined using a variety of techniques, an example of which is an affinity ELISA assay. In various embodiments, affinity is determined by a surface plasmon resonance assay (e.g., BIAcore^{®}-based assay). Using this methodology, the association rate constant (kₐ in M⁻¹s⁻¹) and the dissociation rate constant (k_{d} in s⁻¹) can be measured. The equilibrium dissociation constant (K_{D} in M) can then be calculated from the ratio of the kinetic rate constants (k_{d}/kₐ). In some embodiments, affinity is determined by a kinetic method, such as a Kinetic Exclusion Assay (KinExA) as described in Rathanaswami et al. Analytical Biochemistry, Vol. 373:52-60, 2008. Using a KinExA assay, the equilibrium dissociation constant (K_{D} in M) and the association rate constant (kₐ in M⁻¹s⁻¹) can be measured. The dissociation rate constant (k_{d} in s⁻¹) can be calculated from these values (K_{D} x kₐ). In other embodiments, affinity is determined by an equilibrium/solution method. In certain embodiments, affinity is determined by a FACS binding assay. In certain embodiments of the invention, the antigen binding protein specifically binds to target antigen(s) expressed by a mammalian cell (e.g., CHO, HEK 293, Jurkat), with a K_{D} of 20 nM (2.0 x 10⁻⁸ M) or less, K_{D} of 10 nM (1.0 x 10⁻⁸ M) or less, K_{D} of 1 nM (1.0 x 10⁻⁹ M) or less, K_{D} of 500 pM (5.0 x 10⁻¹⁰ M) or less, K_{D} of 200 pM (2.0 x 10⁻¹⁰ M) or less, K_{D} of 150 pM (1.50 x 10⁻¹⁰ M) or less, K_{D} of 125 pM (1.25 x 10⁻¹⁰ M) or less, K_{D} of 105 pM (1.05 x 10⁻¹⁰ M) or less, K_{D} of 50 pM (5.0 x 10⁻¹¹ M) or less, or K_{D} of 20 pM (2.0 x 10⁻¹¹ M) or less, as determined by a Kinetic Exclusion Assay, conducted by the method described in Rathanaswami et al. Analytical Biochemistry, Vol. 373:52-60, 2008. In some embodiments, the bispecific antigen binding proteins described herein exhibit desirable characteristics such as binding avidity as measured by k_{d} (dissociation rate constant) for target antigen(s) of about 10⁻², 10⁻³, 10⁻⁴, 10⁻⁵, 10⁻⁶, 10⁻⁷, 10⁻⁸, 10⁻⁹, 10⁻¹⁰ s⁻¹ or lower (lower values indicating higher binding avidity), and/or binding affinity as measured by K_{D} (equilibrium dissociation constant) for target antigen(s) of about 10⁻⁹, 10⁻¹⁰, 10⁻¹¹, 10⁻¹², 10⁻¹¹, 10⁻¹⁴, 10⁻¹¹, 10⁻¹⁶ M or lower (lower values indicating higher binding affinity).

In certain embodiments of the invention, the antigen binding proteins are multivalent. The valency of the binding protein denotes the number of individual antigen binding domains within the binding protein. For example, the terms "monovalent," "bivalent," "trivalent", and "tetravalent" with reference to the antigen binding proteins of the invention refer to binding proteins with one, two, and four antigen binding domains, respectively. Thus, a tetravalent antigen binding protein comprises four or more antigen binding domains. In other embodiments, the bispecific antigen binding proteins are multivalent. For instance, in certain embodiments, the bispecific antigen binding proteins are tetravalent comprising four antigen-binding domains: two antigen-binding domains binding to a first target antigen and two antigen-binding domains binding to a second target antigen. A tetraspecific antigen binding protein is tetravalent and comprises four antigen-binding domains: one to antigen-binding domain binding to a first target antigen, one antigen-binding domain binding to a second target antigen, one to antigen-binding domain binding to a third target antigen, and one antigen-binding domain binding to a fourth target antigen.

As used herein, the term "antigen binding domain," which is used interchangeably with "binding domain," refers to the region of the antigen binding protein that contains the amino acid residues that interact with the antigen and confer on the antigen binding protein its specificity and affinity for the antigen. In some embodiments, the binding domain may be derived from the natural ligands of the target antigen(s). As used herein, the term "target antigen(s)" refers to a first target antigen and/or a second target antigen of a bispecific molecule and also refers to a first target antigen, a second target antigen, a third target antigen, and/or a fourth target antigen of a tetraspecific molecule.

In certain embodiments of the antigen binding proteins of the invention, the binding domain may be derived from an antibody or functional fragment thereof. For instance, the binding domains of the antigen binding proteins of the invention may comprise one or more complementarity determining regions (CDR) from the light and heavy chain variable regions of antibodies that specifically bind to target antigen(s). As used herein, the term "CDR" refers to the complementarity determining region (also termed "minimal recognition units" or "hypervariable region") within antibody variable sequences. There are three heavy chain variable region CDRs (CDRH1, CDRH2 and CDRH3) and three light chain variable region CDRs (CDRL1, CDRL2 and CDRL3). The term "CDR region" as used herein refers to a group of three CDRs that occur in a single variable region (i.e. the three-light chain CDRs or the three-heavy chain CDRs). The CDRs in each of the two chains typically are aligned by the framework regions to form a structure that binds specifically with a specific epitope or domain on the target protein. From N-terminus to C-terminus, naturally-occurring light and heavy chain variable regions both typically conform with the following order of these elements: FR1, CDR1, FR2, CDR2, FR3, CDR3 and FR4.

Both the EU index as in Kabat et al., Sequences of Proteins of Immunological Interest, 5th Ed. Public Health Service, National Institutes of Health, Bethesda, MD (1991) and AHo numbering schemes (Honegger A. and Plückthun A. J Mol Biol. 2001 Jun 8;309(3):657-70) can be used in the present invention. Amino acid positions and complementarity determining regions (CDRs) and framework regions (FR) of a given antibody may be identified using either system. For example, EU heavy chain positions of 39, 44, 183, 356, 357, 360, 370, 392, 399, and 409 are equivalent to AHo heavy chain positions 46, 51, 230, 484, 485, 491, 501, 528, 535, and 551, respectively.

Papain digestion of antibodies produces two identical antigen-binding fragments, called "Fab" fragments, each with a single antigen-binding site, and a residual "Fc" fragment which contains the immunoglobulin constant region. The Fab fragment contains all of the variable domain, as well as the constant domain of the light chain and the first constant domain (CH1) of the heavy chain. Thus, a "Fab fragment" is comprised of one immunoglobulin light chain (light chain variable region (VL) and constant region (CL)) and the CH1 region and variable region (VH) of one immunoglobulin heavy chain. The heavy chain of a Fab molecule cannot form a disulfide bond with another heavy chain molecule. The Fc fragment displays carbohydrates and is responsible for many antibody effector functions (such as binding complement and cell receptors), that distinguish one class of antibody from another. The "Fd fragment" comprises the VH and CH1 domains from an immunoglobulin heavy chain. The Fd fragment represents the heavy chain component of the Fab fragment.

A "Fab' fragment" is a Fab fragment having at the C-terminus of the CH1 domain one or more cysteine residues from the antibody hinge region.

A "F(ab')₂ fragment" is a bivalent fragment including two Fab' fragments linked by a disulfide bridge between the heavy chains at the hinge region.

The "Fv" fragment is the minimum fragment that contains a complete antigen recognition and binding site from an antibody. This fragment consists of a dimer of one immunoglobulin heavy chain variable region (VH) and one immunoglobulin light chain variable region (VL) in tight, non-covalent association. It is in this configuration that the three CDRs of each variable region interact to define an antigen binding site on the surface of the VH-VL dimer. A single light chain or heavy chain variable region (or half of an Fv fragment comprising only three CDRs specific for an antigen) has the ability to recognize and bind antigen, although at a lower affinity than the entire binding site comprising both VH and VL.

The "variable region," used interchangeably herein with "variable domain" (variable region of a light chain (VL), variable region of a heavy chain (VH)) refers to the region in each of the light and heavy immunoglobulin chains which is involved directly in binding the antibody to the antigen. As discussed above, the regions of variable light and heavy chains have the same general structure and each region comprises four framework (FR) regions whose sequences are widely conserved, connected by three CDRs. The framework regions adopt a beta-sheet conformation and the CDRs may form loops connecting the beta-sheet structure. The CDRs in each chain are held in their three-dimensional structure by the framework regions and form, together with the CDRs from the other chain, the antigen binding site.

The "immunoglobulin domain" represents a peptide comprising an amino acid sequence similar to that of immunoglobulin and comprising approximately 100 amino acid residues including at least two cysteine residues. Examples of the immunoglobulin domain include VH, CH1, CH2, and CH3 of an immunoglobulin heavy chain, and VL and CL of an immunoglobulin light chain. In addition, the immunoglobulin domain is found in proteins other than immunoglobulin. Examples of the immunoglobulin domain in proteins other than immunoglobulin include an immunoglobulin domain included in a protein belonging to an immunoglobulin super family, such as a major histocompatibility complex (MHC), CD1, B7, T-cell receptor (TCR), and the like. Any of the immunoglobulin domains can be used as an immunoglobulin domain for the multivalent antibody of the present invention.

In a human antibody, CH1 means a region having the amino acid sequence at positions 118 to 215 of the EU index. A highly flexible amino acid region called a "hinge region" exists between CH1 and CH2. CH2 represents a region having the amino acid sequence at positions 231 to 340 of the EU index, and CH3 represents a region having the amino acid sequence at positions 341 to 446 of the EU index.

"CL" represents a constant region of a light chain. In the case of a κ chain of a human antibody, CL represents a region having the amino acid sequence at positions 108 to 214 of the EU index. In a λ chain, CL represents a region having the amino acid sequence at positions 108 to 215.

The binding domains that specifically bind to target antigen(s) can be derived a) from known antibodies to these antigens or b) from new antibodies or antibody fragments obtained by *de novo* immunization methods using the antigen proteins or fragments thereof, by phage display, or other routine methods. The antibodies from which the binding domains for the antigen binding proteins are derived can be monoclonal antibodies, polyclonal antibodies, recombinant antibodies, human antibodies, or humanized antibodies. In certain embodiments, the antibodies from which the binding domains are derived are monoclonal antibodies. In these and other embodiments, the antibodies are human antibodies or humanized antibodies and can be of the IgG1-, IgG2-, IgG3-, or IgG4-type.

As used herein, the terms "stability" and "stabilizing" are defined as the maintenance of the chemical or physical integrity and/or bioactivity of the antigen-binding polypeptide or protein over a period of time. Stabilizing an antigen-binding polypeptide or protein includes the prevention or delay of degradation or deterioration of the antigen- binding polypeptide or protein from its biologically and/or therapeutically active form to an inactive form. Instability may arise from events such as aggregation, denaturation, fragmentation, or chemical modifications such as oxidation, cross- linking, deamidation and reactions with other components featured in the composition comprising the antigen-binding polypeptide or protein.

The stability of the antigen-binding protein or polypeptide in the composition may be characterized using known methods in the art, including but not limited to, measurement of biological activity such as antigen-binding activity with immunoassay techniques such as ELISA, or other techniques of determining purity or physical/chemical changes to the antigen-binding protein or polypeptide such as size exclusion chromatography, capillary gel electrophoresis, circular dichroism, or mass spectrometry. Stability is determined by comparison of measurements obtained via these types of characterization methods at an initial time point, such as at the time of formulation or preparation of the composition (i.e., as the case may be, the suspension or dispersion), and those obtained at a later time point, that is, after storage in a given environment or condition.

The term "monoclonal antibody" (or "mAb") as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against an individual antigenic site or epitope, in contrast to polyclonal antibody preparations that typically include different antibodies directed against different epitopes. Monoclonal antibodies may be produced using any technique known in the art, e.g., by immortalizing spleen cells harvested from the transgenic animal after completion of the immunization schedule. The spleen cells can be immortalized using any technique known in the art, e.g., by fusing them with myeloma cells to produce hybridomas. Myeloma cells for use in hybridoma-producing fusion procedures are non-antibody-producing, have high fusion efficiency, and enzyme deficiencies that render them incapable of growing in certain selective media which support the growth of only the desired fused cells (hybridomas). Examples of suitable cell lines for use in mouse fusions include Sp-20, P3-X63/Ag8, P3-X63-Ag8.653, NS1/1.Ag 4 1, Sp210-Ag14, FO, NSO/U, MPC-11, MPC11-X45-GTG 1.7 and S194/5XXO Bul; examples of cell lines used in rat fusions include R210.RCY3, Y3-Ag 1.2.3, IR983F and 4B210. Other cell lines useful for cell fusions are U-266, GM1500-GRG2, LICR-LON-HMy2 and UC729-6.

In some instances, a hybridoma cell line is produced by immunizing an animal (e.g., a transgenic animal having human immunoglobulin sequences) with a target antigen(s) immunogen; harvesting spleen cells from the immunized animal; fusing the harvested spleen cells to a myeloma cell line, thereby generating hybridoma cells; establishing hybridoma cell lines from the hybridoma cells, and identifying a hybridoma cell line that produces an antibody that binds target antigen(s).

Monoclonal antibodies secreted by a hybridoma cell line can be purified using any technique known in the art, such as protein A-Sepharose, hydroxylapatite chromatography, gel electrophoresis, dialysis, or affinity chromatography. Hybridomas or mAbs may be further screened to identify mAbs with particular properties, such as the ability to bind cells expressing target antigen(s), ability to block or interfere with the binding of target antigen(s) to their respective receptors or ligands, or the ability to functionally block either of target antigen(s).

In some embodiments, the binding domains of the antigen binding proteins of the invention may be derived from humanized antibodies against target antigen(s). A "humanized antibody" refers to an antibody in which regions (e.g. framework regions) have been modified to comprise corresponding regions from a human immunoglobulin. Generally, a humanized antibody can be produced from a monoclonal antibody raised initially in a non-human animal. Certain amino acid residues in this monoclonal antibody, typically from non-antigen recognizing portions of the antibody, are modified to be homologous to corresponding residues in a human antibody of corresponding isotype. Humanization can be performed, for example, using various methods by substituting at least a portion of a rodent variable region for the corresponding regions of a human antibody (see, e.g., United States Patent Nos. 5,585,089 and 5,693,762; Jones et al., Nature, Vol. 321:522-525, 1986; Riechmann et al., Nature, Vol. 332:323-27, 1988; Verhoeyen et al., Science, Vol. 239:1534-1536, 1988). The CDRs of light and heavy chain variable regions of antibodies generated in another species can be grafted to consensus human FRs. To create consensus human FRs, FRs from several human heavy chain or light chain amino acid sequences may be aligned to identify a consensus amino acid sequence.

New antibodies generated against the target antigen(s) from which binding domains for the antigen binding proteins of the invention can be derived can be fully human antibodies. A "fully human antibody" is an antibody that comprises variable and constant regions derived from human germ line immunoglobulin sequences. One specific means provided for implementing the production of fully human antibodies is the "humanization" of the mouse humoral immune system. Introduction of human immunoglobulin (Ig) loci into mice in which the endogenous Ig genes have been inactivated is one means of producing fully human monoclonal antibodies (mAbs) in mouse, an animal that can be immunized with any desirable antigen. Using fully human antibodies can minimize the immunogenic and allergic responses that can sometimes be caused by administering mouse or mouse-derived mAbs to humans as therapeutic agents.

Fully human antibodies can be produced by immunizing transgenic animals (usually mice) that are capable of producing a repertoire of human antibodies in the absence of endogenous immunoglobulin production. Antigens for this purpose typically have six or more contiguous amino acids, and optionally are conjugated to a carrier, such as a hapten. *See, e.g.,* Jakobovits et al., 1993, Proc. Natl. Acad. Sci. USA 90:2551-2555; Jakobovits et al., 1993, Nature 362:255-258; and Bruggermann et al., 1993, Year in Immunol. 7:33. In one example of such a method, transgenic animals are produced by incapacitating the endogenous mouse immunoglobulin loci encoding the mouse heavy and light immunoglobulin chains therein, and inserting into the mouse genome large fragments of human genome DNA containing loci that encode human heavy and light chain proteins. Partially modified animals, which have less than the full complement of human immunoglobulin loci, are then cross-bred to obtain an animal having all of the desired immune system modifications. When administered an immunogen, these transgenic animals produce antibodies that are immunospecific for the immunogen but have human rather than murine amino acid sequences, including the variable regions. For further details of such methods, *see,* for example, WO96/33735 and WO94/02602. Additional methods relating to transgenic mice for making human antibodies are described in United States Patent No. 5,545,807; No. 6,713,610; No. 6,673,986; No. 6,162,963; No. 5,939,598; No. 5,545,807; No. 6,300,129; No. 6,255,458; No. 5,877,397; No. 5,874,299 and No. 5,545,806; in PCT publications WO91/10741, WO90/04036, WO 94/02602, WO 96/30498, WO 98/24893 and in EP 546073B1 and EP 546073A1.

The transgenic mice described above, referred to herein as "HuMab" mice, contain a human immunoglobulin gene minilocus that encodes unrearranged human heavy (mu and gamma) and kappa light chain immunoglobulin sequences, together with targeted mutations that inactivate the endogenous mu and kappa chain loci (Lonberg et al., 1994, Nature 368:856-859). Accordingly, the mice exhibit reduced expression of mouse IgM or kappa and in response to immunization, and the introduced human heavy and light chain transgenes undergo class switching and somatic mutation to generate high affinity human IgG kappa monoclonal antibodies (Lonberg *et al., supra.*; Lonberg and Huszar, 1995, Intern. Rev. Immunol. 13: 65-93; Harding and Lonberg, 1995, Ann. N.Y Acad. Sci. 764:536-546). The preparation of HuMab mice is described in detail in Taylor et al., 1992, Nucleic Acids Research 20:6287-6295; Chen et al., 1993, International Immunology 5:647-656; Tuaillon et al., 1994, J. Immunol. 152:2912-2920; Lonberg et al., 1994, Nature 368:856-859; Lonberg, 1994, Handbook of Exp. Pharmacology 113:49-101; Taylor et al., 1994, International Immunology 6:579-591; Lonberg and Huszar, 1995, Intern. Rev. Immunol. 13:65-93; Harding and Lonberg, 1995, Ann. N.Y Acad. Sci. 764:536-546; Fishwild et al., 1996, Nature Biotechnology 14:845-851; the foregoing references are hereby incorporated by reference in their entirety for all purposes. *See,* further United States Patent No. 5,545,806; No. 5,569,825; No. 5,625,126; No. 5,633,425; No. 5,789,650; No. 5,877,397; No. 5,661,016; No. 5,814,318; No. 5,874,299; and No. 5,770,429; as well as United States Patent No. 5,545,807; International Publication Nos. WO 93/1227; WO 92/22646; and WO 92/03918, the disclosures of all of which are hereby incorporated by reference in their entirety for all purposes. Technologies utilized for producing human antibodies in these transgenic mice are disclosed also in WO 98/24893, and Mendez et al., 1997, Nature Genetics 15:146-156, which are hereby incorporated by reference.

Human-derived antibodies can also be generated using phage display techniques. Phage display is described in e.g., Dower et al., WO 91/17271, McCafferty et al., WO 92/01047, and Caton and Koprowski, Proc. Natl. Acad. Sci. USA, 87:6450-6454 (1990), each of which is incorporated herein by reference in its entirety. The antibodies produced by phage technology are usually produced as antigen binding fragments, e.g. Fv or Fab fragments, in bacteria and thus lack effector functions. Effector functions can be introduced by one of two strategies: The fragments can be engineered either into complete antibodies for expression in mammalian cells, or into antibody fragments with a second binding site capable of triggering an effector function, if desired. Typically, the Fd fragment (VH-CH1) and light chain (VL-CL) of antibodies are separately cloned by PCR and recombined randomly in combinatorial phage display libraries, which can then be selected for binding to a particular antigen. The antibody fragments are expressed on the phage surface, and selection of Fv or Fab (and therefore the phage containing the DNA encoding the antibody fragment) by antigen binding is accomplished through several rounds of antigen binding and re-amplification, a procedure termed panning. Antibody fragments specific for the antigen are enriched and finally isolated. Phage display techniques can also be used in an approach for the humanization of rodent monoclonal antibodies, called "guided selection" (see Jespers, L. S., et al., Bio/Technology 12, 899-903 (1994)). For this, the Fd fragment of the mouse monoclonal antibody can be displayed in combination with a human light chain library, and the resulting hybrid Fab library may then be selected with antigen. The mouse Fd fragment thereby provides a template to guide the selection. Subsequently, the selected human light chains are combined with a human Fd fragment library. Selection of the resulting library yields entirely human Fab.

In certain embodiments, the antigen binding proteins of the invention comprise antibodies. As used herein, the term "antibody" refers to a tetrameric immunoglobulin protein comprising two light chain polypeptides (about 25 kDa each) and two heavy chain polypeptides (about 50-70 kDa each). The term "light chain" or "immunoglobulin light chain" refers to a polypeptide comprising, from amino terminus to carboxyl terminus, a single immunoglobulin light chain variable region (VL) and a single immunoglobulin light chain constant domain (CL). The immunoglobulin light chain constant domain (CL) can be kappa (κ) or lambda (λ).The term "heavy chain" or "immunoglobulin heavy chain" refers to a polypeptide comprising, from amino terminus to carboxyl terminus, a single immunoglobulin heavy chain variable region (VH), an immunoglobulin heavy chain constant domain 1 (CH1), an immunoglobulin hinge region, an immunoglobulin heavy chain constant domain 2 (CH2), an immunoglobulin heavy chain constant domain 3 (CH3), and optionally an immunoglobulin heavy chain constant domain 4 (CH4). Heavy chains are classified as mu (µ), delta (Δ), gamma (γ), alpha (α), and epsilon (ε), and define the antibody's isotype as IgM, IgD, IgG, IgA, and IgE, respectively. The IgG-class and IgA-class antibodies are further divided into subclasses, namely, IgG1, IgG2, IgG3, and IgG4, and IgA1 and IgA2, respectively. The heavy chains in IgG, IgA, and IgD antibodies have three domains (CH1, CH2, and CH3), whereas the heavy chains in IgM and IgE antibodies have four domains (CH1, CH2, CH3, and CH4). The immunoglobulin heavy chain constant domains can be from any immunoglobulin isotype, including subtypes. The antibody chains are linked together via inter-polypeptide disulfide bonds between the CL domain and the CH1 domain (i.e. between the light and heavy chain) and between the hinge regions of the antibody heavy chains.

In particular embodiments, the antigen binding proteins of the invention are heterodimeric antibodies (used interchangeably herein with "hetero immunoglobulins" or "hetero Igs"), which refer to antibodies comprising two different light chains and two different heavy chains.

The heterodimeric antibodies can comprise any immunoglobulin constant region. The term "constant region" as used herein refers to all domains of an antibody other than the variable region. The constant region is not involved directly in binding of an antigen, but exhibits various effector functions. As described above, antibodies are divided into particular isotypes (IgA, IgD, IgE, IgG, and IgM) and subtypes (IgG1, IgG2, IgG3, IgG4, IgA1 IgA2) depending on the amino acid sequence of the constant region of their heavy chains. The light chain constant region can be, for example, a kappa- or lambda-type light chain constant region, e.g., a human kappa- or lambda-type light chain constant region, which are found in all five antibody isotypes. Examples of human immunoglobulin light chain constant region sequences are shown in the following Table 1.

**Table 1. Exemplary Human Immunoglobulin Light Chain Constant Regions**

| **Designation** | **SEQ ID NO:** | **CL Domain Amino Acid Sequence** |
|---|---|---|
| CL-1 | 1 | GQPKANPTVTLFPPSSEELQANKATLVCLISDFYPGAVTVAWKA DGSPVKAGVETTKPSKQSNNKYAASSYLSLTPEQWKSHRSYSC QVTHEGSTVEKTVAPTECS |
| CL-2 | 2 | GQPKAAPSVTLFPPSSEELQANKATLVCLISDFYPGAVTVAWKA DSSPVKAGVETTTPSKQSNNKYAASSYLSLTPEQWKSHRSYSCQ VTHEGSTVEKTVAPTECS |
| CL-3 | 3 | GQPKAAPSVTLFPPSSEELQANKATLVCLISDFYPGAVTVAWKA DSSPVKAGVETTTPSKQSNNKYAASSYLSLTPEQWKSHKSYSCQ VTHEGSTVEKTVAPTECS |
| CL-7 | 4 | GQPKAAPSVTLFPPSSEELQANKATLVCLVSDFYPGAVTVAWK ADGSPVKVGVETTKPSKQSNNKYAASSYLSLTPEQWKSHRSYS CRVTHEGSTVEKTVAPAECS |

The heavy chain constant region of the heterodimeric antibodies can be, for example, an alpha-, delta-, epsilon-, gamma-, or mu-type heavy chain constant region, e.g., a human alpha-, delta-, epsilon-, gamma-, or mu-type heavy chain constant region. In some embodiments, the heterodimeric antibodies comprise a heavy chain constant region from an IgG1, IgG2, IgG3, or IgG4 immunoglobulin. In one embodiment, the heterodimeric antibody comprises a heavy chain constant region from a human IgG1 immunoglobulin. In another embodiment, the heterodimeric antibody comprises a heavy chain constant region from a human IgG2 immunoglobulin. Examples of human IgG1 and IgG2 heavy chain constant region sequences are shown below in Table 2.

**Table 2. Exemplary Human Immunoglobulin Heavy Chain Constant Regions**

| **Ig isotype** | **SEQ ID NO:** | **Heavy Chain Constant Region Amino Acid Sequence** |
|---|---|---|
| Human IgG1z | 5 | ASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPCP APELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAK GQPREPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGK |
| Human IgG1za | 6 | |
| Human IgG1f | 7 | ASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKRVEPKSCDKTHTCPPCP APELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAK GQPREPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGK |
| Human IgG1fa | 8 | ASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKRVEPKSCDKTHTCPPCP APELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPGK |
| Human IgG2 | 9 | ASTKGPSVFPLAPCSRSTSESTAALGCLVKDYFPEPVTVSWNSGALTSGVHTFPAV LQSSGLYSLSSVVTVPSSNFGTQTYTCNVDHKPSNTKVDKTVERKCCVECPPCPAP PVAGPSVFLFPPKPKDTLMISRTPEVTCVVVDVSHEDPEVQFNWYVDGVEVHNAK TKPREEQFNSTFRVVSVLTVVHQDWLNGKEYKCKVSNKGLPAPIEKTISKTKGQPR EPQVYTLPPSREEMTKNQVSLTCLVKGFYPSDIAVEWESNGQPENNYKTTPPMLDS DGSFFLYSKLTVDKSRWQQGNVFSCSVMHEALHNHYTQKSLSLSPGK |

A variable region may be attached to the above light and heavy chain constant regions to form complete antibody light and heavy chains, respectively. Further, each of the so generated heavy and light chain polypeptides may be combined to form a complete bispecific antibody structure, e.g. a heterodimeric antibody. It should be understood that the heavy chain and light chain variable regions provided herein can also be attached to other constant domains having different sequences than the exemplary sequences listed above.

In certain embodiments of the invention two different heavy chains are used to form the a heterodimeric molecule of the present invention. To facilitate assembly of the light and heavy chains from into a heterodimeric antibody, the light chains and/or heavy chains from each antibody can be engineered to reduce the formation of mispaired molecules. For example, one approach to promote heterodimer formation over homodimer formation is the so-called "knobs-into-holes" method, which involves introducing mutations into the CH3 domains of two different antibody heavy chains at the contact interface. Specifically, one or more bulky amino acids in one heavy chain are replaced with amino acids having short side chains (e.g. alanine or threonine) to create a "hole," whereas one or more amino acids with large side chains (e.g. tyrosine or tryptophan) are introduced into the other heavy chain to create a "knob." When the modified heavy chains are co-expressed, a greater percentage of heterodimers (knob-hole) are formed as compared to homodimers (hole-hole or knob-knob). The "knobs-into-holes" methodology is described in detail in WO 96/027011; Ridgway et al., Protein Eng., Vol. 9: 617-621, 1996; and Merchant et al., Nat, Biotechnol., Vol. 16: 677-681, 1998, all of which are hereby incorporated by reference in their entireties.

Another approach for promoting heterodimer formation to the exclusion of homodimer formation entails utilizing an electrostatic steering mechanism (*see* Gunasekaran et al., J. Biol. Chem., Vol. 285: 19637-19646, 2010, which is hereby incorporated by reference in its entirety). This approach involves introducing or exploiting charged residues in the CH3 domain in each heavy chain so that the two different heavy chains associate through opposite charges that cause electrostatic attraction. Homodimerization of the identical heavy chains are disfavored because the identical heavy chains have the same charge and therefore are repelled. This same electrostatic steering technique can be used to prevent mispairing of light chains with the non-cognate heavy chains by introducing residues having opposite charges in the correct light chain - heavy chain pair at the binding interface. The electrostatic steering technique and suitable charge pair mutations for promoting heterodimers and correct light chain/heavy chain pairing is described in WO2009089004 and WO2014081955, both of which are hereby incorporated by reference in their entireties.

In embodiments in which the bispecific antigen binding proteins of the invention are heterodimeric antibodies comprising a first light chain (LC1) and first heavy chain (HC1) from a first antibody that specifically binds to a first target antigen and a second light chain (LC2) and second heavy chain (HC2) from a second antibody that specifically binds to target 2, HC1 or HC2 may comprise one or more amino acid substitutions to replace a positively-charged amino acid with a negatively-charged amino acid. For instance, in one embodiment, the CH3 domain of HC1 or the CH3 domain of HC2 comprises an amino acid sequence differing from a wild-type IgG amino acid sequence such that one or more positively-charged amino acids (e.g., lysine, histidine and arginine) in the wild-type human IgG amino acid sequence are replaced with one or more negatively-charged amino acids (e.g., aspartic acid and glutamic acid) at the corresponding position(s) in the CH3 domain. In these and other embodiments, amino acids (e.g. lysine) at one or more positions selected from 370, 392 and 409 (EU numbering system) are replaced with a negatively-charged amino acid (e.g., aspartic acid and glutamic acid). An amino acid substitution in an amino acid sequence is typically designated herein with a one-letter abbreviation for the amino acid residue in a particular position, followed by the numerical amino acid position relative to an original sequence of interest, which is then followed by the one-letter symbol for the amino acid residue substituted in. For example, "T30D" symbolizes a substitution of a threonine residue by an aspartate residue at amino acid position 30, relative to the original sequence of interest. Another example, "S218G" symbolizes a substitution of a serine residue by a glycine residue at amino acid position 218, relative to the original amino acid sequence of interest.

In certain embodiments, HC1 or HC2 of the heterodimeric antibodies may comprise one or more amino acid substitutions to replace a negatively-charged amino acid with a positively-charged amino acid. For instance, in one embodiment, the CH3 domain of HC1 or the CH3 domain of HC2 comprises an amino acid sequence differing from wild-type IgG amino acid sequence such that one or more negatively-charged amino acids in the wild-type human IgG amino acid sequence are replaced with one or more positively-charged amino acids at the corresponding position(s) in the CH3 domain. In these and other embodiments, amino acids (e.g., aspartic acid or glutamic acid) at one or more positions selected from 356, 357, and 399 (EU numbering system) of the CH3 domain are replaced with a positively-charged amino acid (e.g., lysine, histidine and arginine).

In particular embodiments, the heterodimeric antibody comprises a first heavy chain comprising negatively-charged amino acids at positions 360, 370, 392 and 409 (e.g., K360E/D, 370E/D, K392E/D and K409E/D substitutions), and a second heavy chain comprising positively-charged amino acids at positions 356 and 399 (e.g., E356K and D399K substitutions). In other particular embodiments, the heterodimeric antibody comprises a first heavy chain comprising negatively-charged amino acids at positions 392, 409, and 370 (e.g., K392D, K409D, and K370D substitutions), and a second heavy chain comprising positively-charged amino acids at positions 356, 399, and 357 (e.g., E356K, D399K, and E357K substitutions). In related embodiments, the first heavy chain is from an anti-first target antigen antibody and the second heavy chain is from an anti-second target antigen antibody.

The present invention is directed to a an isolated heteromultimer comprising a heterodimeric CH3 domain comprising a first CH3 domain polypeptide and a second CH3 domain polypeptide, the first CH3 domain polypeptide comprising an amino acid modification at position K360, wherein (i) the first CH3 domain polypeptide further comprises an amino acid modification at position K370, and (ii) the second CH3 domain polypeptide comprises an amino acid modification at position E357, wherein the numbering of amino acid residues is according to the EU index as set forth in Kabat.

In another aspect the present invention is directed to a method of stabilizing an isolated heteromultimer at about pH 5.0 wherein the heteromultimer comprises a heterodimeric CH3 domain comprising a first CH3 domain polypeptide and a second CH3 domain polypeptide, wherein: (i) the first CH3 domain polypeptide comprises an amino acid modification at position K370, and (ii) the second CH3 domain polypeptide comprises an amino acid modification at position E357, the method comprising introducing an amino acid modification at position K360 of the fist CH3 domain, wherein the modification is substitution of K360 for a glutamic or aspartic acid; wherein the numbering of amino acid residues is according to the EU index as set forth in Kabat.

In one embodiment the amino acid modification at position K360 is selected from the group consisting of K360E and K360D.

In one embodiment amino acid modification at position K370 is selected from the group consisting of K370E and K370D.

In one embodiment the amino acid modification at position E357 is selected from the group consisting of E357K, E357H, and E357R.

In one embodiment the amino acid modification at position K360 is K360E, the amino acid modification at position K370 is K370D, and the amino acid modification at position E357 is E357K.

In one embodiment one CH3 domain polypeptide further comprises an amino acid modification at position K409, and the other CH3 domain polypeptide further comprises an amino acid modification at position D399.

In one embodiment the first CH3 domain polypeptide comprises the amino acid modification at position K409, and the second CH3 domain polypeptide comprises the amino acid modification at position D399.

In one embodiment the first CH3 domain polypeptide comprises the amino acid modification at position D399, and the second CH3 domain polypeptide comprises the amino acid modification at position K409.

In one embodiment the amino acid modification at position K409 is selected from the group consisting of K409E and K409D, and wherein the amino acid modification at position D399 is selected from the group consisting of D399K, D399H, and D399R.

In one embodiment the amino acid modification at position K409 is K409D, and wherein the amino acid modification at position D399 is D399K.

In one embodiment the CH3 domain polypeptide which comprises the amino acid modification at position K409, further comprises an amino acid modification at position K392.

In one embodiment the amino acid modification at position K392 is selected from the group consisting of K392E and K392D.

In one embodiment one CH3 domain polypeptide further comprises an amino acid modification at position K439, and the other CH3 domain polypeptide further comprises an amino acid modification at position E356.

In one embodiment the first CH3 domain polypeptide comprises the amino acid modification at position K439, and the second CH3 domain polypeptide comprises the amino acid modification at position E356.

In one embodiment the first CH3 domain polypeptide comprises the amino acid modification at position E356, and the second CH3 domain polypeptide comprises the amino acid modification at position K439.

In one embodiment the amino acid modification at position K439 is selected from the group consisting of K439E and K439D, and wherein the amino acid modification at position E356 is selected from the group consisting of E356K, E356H, and E356R.

In one embodiment the amino acid modification at position K439 is K439E, and wherein the amino acid modification at position E356 is E356K.

In one embodiment the first CH3 domain polypeptide comprises K360E, K370D, K409, and K392D mutations and second CH3 domain polypeptide comprises E357K and D399K mutations.

In one embodiment the first CH3 domain polypeptide further comprises a K439E mutation, and the second CH3 domain polypeptide further comprises a E356K mutation.

In one embodiment the heterodimeric CH3 domain is comprised by an Fc region based on an IgG Fc region.

In one embodiment the IgG Fc region is an IgG1 Fc region.

In one embodiment the heteromultimer is a bispecific or multispecific antibody.

To facilitate the association of a particular heavy chain with its cognate light chain, both the heavy and light chains may contain complimentary amino acid substitutions. As used herein, "complimentary amino acid substitutions" refer to a substitution to a positively-charged amino acid in one chain paired with a negatively-charged amino acid substitution in the other chain. For example, in some embodiments, the heavy chain comprises at least one amino acid substitution to introduce a charged amino acid and the corresponding light chain comprises at least one amino acid substitution to introduce a charged amino acid, wherein the charged amino acid introduced into the heavy chain has the opposite charge of the amino acid introduced into the light chain. In certain embodiments, one or more positively-charged residues (e.g., lysine, histidine or arginine) can be introduced into a first light chain (LC1) and one or more negatively-charged residues (e.g., aspartic acid or glutamic acid) can be introduced into the companion heavy chain (HC1) at the binding interface of LC1/HC1, whereas one or more negatively-charged residues (e.g., aspartic acid or glutamic acid) can be introduced into a second light chain (LC2) and one or more positively-charged residues (e.g., lysine, histidine or arginine) can be introduced into the companion heavy chain (HC2) at the binding interface of LC2/HC2. The electrostatic interactions will direct the LC1 to pair with HC1 and LC2 to pair with HC2, as the opposite charged residues (polarity) at the interface attract. The heavy/light chain pairs having the same charged residues (polarity) at an interface (e.g. LC1/HC2 and LC2/HC1) will repel, resulting in suppression of the unwanted HC/LC pairings.

In these and other embodiments, the CH1 domain of the heavy chain or the CL domain of the light chain comprises an amino acid sequence differing from wild-type IgG amino acid sequence such that one or more positively-charged amino acids in wild-type IgG amino acid sequence is replaced with one or more negatively-charged amino acids. Alternatively, the CH1 domain of the heavy chain or the CL domain of the light chain comprises an amino acid sequence differing from wild-type IgG amino acid sequence such that one or more negatively-charged amino acids in wild-type IgG amino acid sequence is replaced with one or more positively-charged amino acids. In some embodiments, one or more amino acids in the CH1 domain of the first and/or second heavy chain in the heterodimeric antibody at an EU position selected from F126, P127, L128, A141, L145, K147, D148, H168, F170, P171, V173, Q175, S176, S183, V185 and K213 is replaced with a charged amino acid. In certain embodiments, a heavy chain residue for substitution with a negatively- or positively- charged amino acid is S183 (EU numbering system). In some embodiments, S183 is substituted with a positively-charged amino acid. In alternative embodiments, S183 is substituted with a negatively-charged amino acid. For instance, in one embodiment, S183 is substituted with a negatively-charged amino acid (e.g. S183E) in the first heavy chain, and S183 is substituted with a positively-charged amino acid (e.g. S183K) in the second heavy chain.

In embodiments in which the light chain is a kappa light chain, one or more amino acids in the CL domain of the first and/or second light chain in the heterodimeric antibody at a position (EU numbering in a kappa light chain) selected from F116, F118, S121, D122, E123, Q124, S131, V133, L135, N137, N138, Q160, S162, T164, S174 and S176 is replaced with a charged amino acid. In embodiments in which the light chain is a lambda light chain, one or more amino acids in the CL domain of the first and/or second light chain in the heterodimeric antibody at a position (EU numbering in a lambda chain) selected from T116, F118, S121, E123, E124, K129, T131, V133, L135, S137, E160, T162, S165, Q167, A174, S176 and Y178 is replaced with a charged amino acid. In some embodiments, a residue for substitution with a negatively- or positively- charged amino acid is S176 (EU numbering system) of the CL domain of either a kappa or lambda light chain. In certain embodiments, S176 of the CL domain is replaced with a positively-charged amino acid. In alternative embodiments, S176 of the CL domain is replaced with a negatively-charged amino acid. In one embodiment, S176 is substituted with a positively-charged amino acid (e.g. S176K) in the first light chain, and S176 is substituted with a negatively-charged amino acid (e.g. S176E) in the second light chain.

In addition to or as an alternative to the complimentary amino acid substitutions in the CH1 and CL domains, the variable regions of the light and heavy chains in the heterodimeric antibody may contain one or more complimentary amino acid substitutions to introduce charged amino acids. For instance, in some embodiments, the VH region of the heavy chain or the VL region of the light chain of a heterodimeric antibody comprises an amino acid sequence differing from wild-type IgG amino acid sequence such that one or more positively-charged amino acids in wild-type IgG amino acid sequence is replaced with one or more negatively-charged amino acids. Alternatively, the VH region of the heavy chain or the VL region of the light chain comprises an amino acid sequence differing from wild-type IgG amino acid sequence such that one or more negatively-charged amino acids in wild-type IgG amino acid sequence is replaced with one or more positively-charged amino acids.

V region interface residues (i.e., amino acid residues that mediate assembly of the VH and VL regions) within the VH region include EU positions 1, 3, 35, 37, 39, 43, 44, 45, 46, 47, 50, 59, 89, 91, and 93. One or more of these interface residues in the VH region can be substituted with a charged (positively- or negatively-charged) amino acid. In certain embodiments, the amino acid at EU position 39 in the VH region of the first and/or second heavy chain is substituted for a positively-charged amino acid, e.g., lysine. In alternative embodiments, the amino acid at EU position 39 in the VH region of the first and/or second heavy chain is substituted for a negatively-charged amino acid, e.g., glutamic acid. In some embodiments, the amino acid at EU position 39 in the VH region of the first heavy chain is substituted for a negatively-charged amino acid (e.g. G39E), and the amino acid at EU position 39 in the VH region of the second heavy chain is substituted for a positively-charged amino acid (e.g. G39K). In some embodiments, the amino acid at EU position 44 in the VH region of the first and/or second heavy chain is substituted for a positively-charged amino acid, e.g., lysine. In alternative embodiments, the amino acid at EU position 44 in the VH region of the first and/or second heavy chain is substituted for a negatively-charged amino acid, e.g., glutamic acid. In certain embodiments, the amino acid at EU position 44 in the VH region of the first heavy chain is substituted for a negatively-charged amino acid (e.g. G44E), and the amino acid at EU position 44 in the VH region of the second heavy chain is substituted for a positively-charged amino acid (e.g. G44K).

V region interface residues (i.e., amino acid residues that mediate assembly of the VH and VL regions) within the VL region include EU positions 32, 34, 35, 36, 38, 41, 42, 43, 44, 45, 46, 48, 49, 50, 51, 53, 54, 55, 56, 57, 58, 85, 87, 89, 90, 91, and 100. One or more interface residues in the VL region can be substituted with a charged amino acid, preferably an amino acid that has an opposite charge to those introduced into the VH region of the cognate heavy chain. In some embodiments, the amino acid at EU position 100 in the VL region of the first and/or second light chain is substituted for a positively-charged amino acid, e.g., lysine. In alternative embodiments, the amino acid at EU position 100 in the VL region of the first and/or second light chain is substituted for a negative-charged amino acid, e.g., glutamic acid. In certain embodiments, the amino acid at EU position 100 in the VL region of the first light chain is substituted for a positively-charged amino acid (e.g. G100K), and the amino acid at EU position 100 in the VL region of the second light chain is substituted for a negatively-charged amino acid (e.g. G100E).

Any of the constant domains can be modified to contain one or more of the charge pair mutations described above to facilitate correct assembly of a heterodimeric antibody.

The inventive heterodimeric antibodies also encompass antibodies comprising the heavy chain(s) and/or light chain(s), where one, two, three, four or five amino acid residues are lacking from the N-terminus or C-terminus, or both, in relation to any one of the heavy and light chains, e.g., due to post-translational modifications resulting from the type of host cell in which the antibodies are expressed. For instance, Chinese Hamster Ovary (CHO) cells frequently cleave off a C-terminal lysine from antibody heavy chains.

In certain embodiments, the antigen binding proteins of the invention comprise (i) a first binding domain that specifically binds a first target antigen, (ii) a second binding domain that specifically binds to a second target antigen, and (iii) a human immunoglobulin Fc region, wherein one of the binding domains is positioned at the amino terminus of the Fc region and the other binding domain is positioned at the carboxyl terminus of the Fc region. In some such embodiments, each of the first and second binding domains comprises immunoglobulin variable regions. For instance, in certain embodiments, the first binding domain comprises a first light chain variable region (VL1) and a first heavy chain variable region (VH1) from an anti-first target antigen antibody and the second binding domain comprises a second light chain variable region (VL2) and a second heavy chain variable region (VH2) from an anti-second target antigen antibody.

As used herein, the term "Fc region" refers to the C-terminal region of an immunoglobulin heavy chain which may be generated by papain digestion of an intact antibody. The Fc region of an immunoglobulin generally comprises two constant domains, a CH2 domain and a CH3 domain, and optionally comprises a CH4 domain. In certain embodiments, the Fc region is an Fc region from an IgG1, IgG2, IgG3, or IgG4 immunoglobulin. In some embodiments, the Fc region comprises CH2 and CH3 domains from a human IgG1 or human IgG2 immunoglobulin. The Fc region may retain effector function, such as C1q binding, complement dependent cytotoxicity (CDC), Fc receptor binding, antibody-dependent cell-mediated cytotoxicity (ADCC), and phagocytosis. In other embodiments, the Fc region may be modified to reduce or eliminate effector function as described in further detail herein.

In certain embodiments of the bispecific antigen binding proteins of the invention, the binding domain positioned at the amino terminus of the Fc region (i.e. the amino-terminal binding domain) is a Fab fragment fused to the amino terminus of the Fc region through a peptide linker described herein or through an immunoglobulin hinge region. An "immunoglobulin hinge region" refers to the amino acid sequence connecting the CH1 domain and the CH2 domain of an immunoglobulin heavy chain. The hinge region of human IgG1 is generally defined as the amino acid sequence from about Glu216 or about Cys226, to about Pro230. Hinge regions of other IgG isotypes may be aligned with the IgG1 sequence by placing the first and last cysteine residues forming inter-heavy chain disulfide bonds in the same positions and are determinable to those of skill in the art. In some embodiments, the amino-terminal binding domain is joined to the amino terminus of the Fc region through a human IgG1 hinge region. In other embodiments, the amino-terminal binding domain is joined to the amino terminus of the Fc region through a human IgG2 hinge region. In one embodiment, the amino-terminal binding domain (e.g. Fab fragment) is fused to the Fc region through the carboxyl terminus of the CH1 region of the Fab.

As used herein, the term "modified heavy chain" refers to a fusion protein comprising an immunoglobulin heavy chain, particularly a human IgG1 or human IgG2 heavy chain, and a functional antibody fragment (e.g. Fab) or portion thereof (e.g. immunoglobulin light chain or Fd fragment), wherein the fragment or portion thereof is fused at its N-terminus, optionally through a peptide linker, to the C-terminus of the heavy chain.

In some embodiments of the antigen binding proteins of the invention, the binding domain positioned at the carboxyl terminus of the Fc region (i.e. the carboxyl-terminal binding domain) is a Fab fragment. In such embodiments, the Fab is fused or otherwise connected to the carboxyl terminus of the Fc region (e.g. the carboxyl terminus of the CH3 domain) through a peptide linker through the amino terminus of the VH region of the Fab fragment. Thus, in one embodiment, the Fab is fused to an Fc region through the amino terminus of the VH region of the Fab such that the resulting fusion protein comprises, from N-terminus to C-terminus, a CH2 domain, a CH3 domain, a peptide linker, a VH region, and a CH1 region.

The peptide linker joining the Fc region to the carboxyl-terminal Fab can be any of the peptide linkers described herein. In particular embodiments, the peptide linker joining the Fc region to the carboxyl-terminal Fab fragment is at least 5 amino acids in length. In other embodiments, the peptide linker joining the Fc region to the carboxyl-terminal Fab fragment is at least 8 amino acids in length. Particularly suitable peptide linkers for joining the Fc region to the carboxyl-terminal Fab fragment are glycine-serine linkers, such as (GlyₓSer)ₙ where x=3 or 4 and n= 2, 3, 4, 5 or 6. In one embodiment, the peptide linker connecting the Fc region to the carboxyl-terminal Fab fragment is a L10 (G₄S)₂ linker (SEQ ID NO: 10). In another embodiment, the peptide linker connecting the Fc region to the carboxyl-terminal Fab fragment is a L9 or G₃SG₄S linker (SEQ ID NO: 11).

In some embodiments of the antigen binding proteins of the invention in which the carboxyl-terminal binding domain is a Fab fragment, the binding domain positioned at the amino terminus of the Fc region (i.e. the amino-terminal binding domain) is also a Fab fragment. The amino-terminal Fab fragment can be fused to the amino terminus of the Fc region through a peptide linker or an immunoglobulin hinge region described herein. In some embodiments, the amino-terminal Fab fragment is joined to the amino terminus of the Fc region through a human IgG1 hinge region. In other embodiments, the amino-terminal Fab fragment is joined to the amino terminus of the Fc region through a human IgG2 hinge region. In one embodiment, the amino-terminal Fab fragment is fused to the Fc region through the carboxyl terminus of the CH1 region of the Fab.

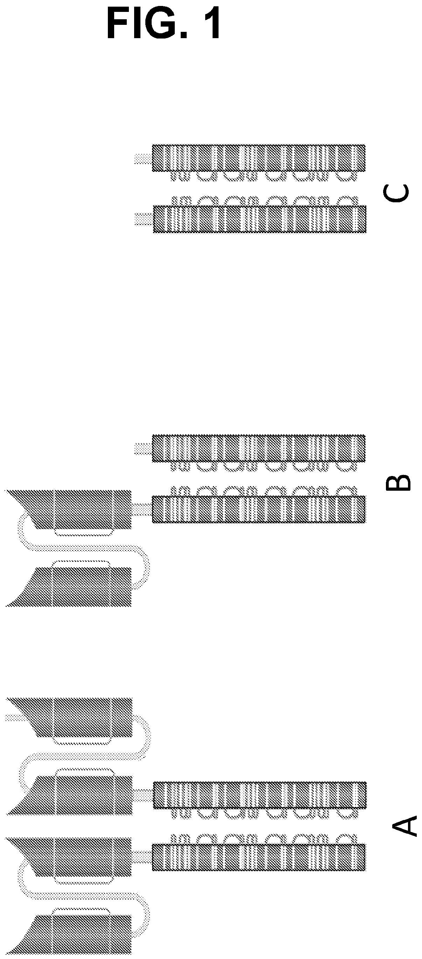

In some embodiments, the bispecific antigen binding protein of the invention comprises a first antibody that specifically binds to a first target where one polypeptide chain (e.g. the heavy chain (VH2-CH1)) of a Fab fragment from a second antibody that specifically binds to a second target is fused to the carboxyl terminus of the heavy chain of the first antibody. The bispecific antigen binding protein in such embodiments also comprises a polypeptide chain containing the other half of the Fab fragment from the second antibody (e.g., the light chain (VL2-CL)). This format is referred to herein as the "IgG-Fab" format, and one embodiment of this type of molecule is shown schematically in Figure 1. Thus, in certain embodiments, the present invention includes a bispecific, multivalent antigen binding protein comprising: (i) a light chain from a first antibody, (ii) a heavy chain from the first antibody, wherein the heavy chain is fused at its carboxyl terminus through a peptide linker to a first polypeptide comprising VH-CH1 domains of a second antibody to form a modified heavy chain, and (iii) a second polypeptide comprising VL-CL domains of the second antibody. When dimerized, the bispecific antigen binding protein is a homohexamer comprising two modified heavy chains, two light chains from the first antibody, and two polypeptide chains containing the other half of the Fab fragment from the second antibody (the Fd fragment). In one embodiment, the first polypeptide, which is fused to the carboxyl terminus of the heavy chain, comprises VH and CH1 domains from the second antibody, and the second polypeptide comprises VL and CL domains from the second antibody.

Charge pair mutations or complimentary amino acid substitutions as described herein can be introduced into the Fab regions of the first antibody (Fab 1) or second antibody (Fab 2) to promote correct heavy chain-light chain pairing. For instance, in some embodiments, the amino acid at EU position 38 of the VL domain in Fab 1 is replaced with a negatively-charged amino acid (e.g. glutamic acid) and the amino acid at EU position 39 of the VH domain in Fab 1 is replaced with a positively-charged amino acid (e.g. lysine). In other embodiments, the amino acid at EU position 38 of the VL domain in Fab 1 is replaced with a positively-charged amino acid (e.g. lysine) and the amino acid at EU position 39 of the VH domain in Fab 1 is replaced with a negatively-charged amino acid (e.g. glutamic acid). In certain embodiments, the amino acid at EU position 38 of the VL domain in Fab 2 is replaced with a negatively-charged amino acid (e.g. glutamic acid) and the amino acid at EU position 39 of the VH domain in Fab 2 is replaced with a positively-charged amino acid (e.g. lysine). In other embodiments, the amino acid at EU position 38 of the VL domain in Fab 2 is replaced with a positively-charged amino acid (e.g. lysine) and the amino acid at EU position 39 of the VH domain in Fab 2 is replaced with a negatively-charged amino acid (e.g. glutamic acid).

In embodiments in which the VH-CH1 region (i.e. Fd fragment) from the second antibody is fused to the heavy chain of the first antibody, the heavy chain from the first antibody comprises a S183E mutation (EU numbering), the light chain from the first antibody comprises a S176K mutation (EU numbering), the light chain from the second antibody comprises a S176E mutation (EU numbering), and the Fd region from the second antibody (which is fused to the C-terminus of the heavy chain from the first antibody) comprises a S183K mutation (EU numbering). In other embodiments, the heavy chain from the first antibody comprises a G44E mutation (EU) and S183E mutation (EU numbering), the light chain from the first antibody comprises a G100K mutation (EU) and S176K mutation (EU numbering), the light chain from the second antibody comprises a G100E mutation (EU) and S176E mutation (EU numbering), and the Fd region from the second antibody (which is fused to the C-terminus of the heavy chain from the first antibody) comprises a G44K mutation (EU) and S183K mutation (EU numbering). The charges in the foregoing examples may be reversed so long as the charge on the corresponding light or heavy chain is also reversed so that the correct heavy/light chain pairs have opposite charges.

"Corresponds to" as it pertains to the VH2 and second CH1 domain means that the amino acid residues of the VH2 and second CH1 domain are counted from the C-terminus of the first heavy chain if there is no linker. If there is a peptide linker, the amino acid residues of the VH2 and second CH1 domain are counted from the C-terminus of the peptide linker. In neither case are the amino acid residues counted from the N-terminus of the first heavy chain. Rather, for the VH2 and second CH1 domain, counting begins at the first amino acid residue of the VH2 domain. The counting of amino acid residues is performed using the EU or AHo convention.

In certain embodiments: a) the VH1 comprises a Q39E mutation and the first CH1 domain comprises a S183K mutation using EU numbering; b) the VH2 comprises a Q39K mutation and the second CH1 domain comprises a S183E mutation using EU numbering; c) the VL1 comprises a Q38K mutation and the first CL domain comprises a S176E mutation using EU numbering; and d) the VL2 comprises a Q38E mutation and the second CL domain comprises a S176K mutation using EU numbering.

In certain embodiments: a) the first CH1 domain comprises G44E and S183K mutations using EU numbering; b) the second CH1 domain comprises G44K and S183E mutations using EU numbering; c) the first CL domain comprises G100K and S176E mutations using EU numbering; and d) the second CL domain comprises G100E and S176K mutations using EU numbering.

In certain embodiments: a) the VH1 comprises a Q39K mutation and the first CH1 domain comprises a S183E mutation using EU numbering; b) the VH2 comprises a Q39E mutation and the second CH1 domain comprises a S183K mutation using EU numbering; c) the VL1 comprises a Q38E mutation and the first CL domain comprises a S176K mutation using EU numbering; and d) the VL2 comprises a Q38K mutation and the second CL domain comprises a S176E mutation using EU numbering.

In certain embodiments: a) the first CH1 domain comprises G44K and S183E mutations using EU numbering; b) the second CH1 domain comprises G44E and S183K mutations using EU numbering; c) the first CL domain comprises G100E and S176K mutations using EU numbering; and d) the second CL domain comprises G100K and S176E mutations using EU numbering.

In certain embodiments the first heavy chain is fused to the VH2 via a peptide linker. In certain embodiments the peptide linker comprises a sequence selected from the group consisting of (Gly₃Ser)₂, (Gly₄Ser)₂, (Gly₃Ser)₃, (Gly₄Ser)₃, (Gly₃Ser)₄, (Gly₄Ser)₄, (Gly₃Ser)₅, (Gly₄Ser)₅, (Gly₃Ser)₆, and (Gly₄Ser)₆. These sequences can also be written as GGGSGGGS (SEQ ID NO: 12), GGGGSGGGGS (SEQ ID NO: 13), GGGSGGGSGGGS (SEQ ID NO: 14), GGGGSGGGGSGGGGS (SEQ ID NO: 15), GGGSGGGSGGGSGGGS (SEQ ID NO: 16), GGGGSGGGGSGGGGSGGGGS (SEQ ID NO: 17), GGGSGGGSGGGSGGGSGGGS (SEQ ID NO: 18), GGGGSGGGGSGGGGSGGGGSGGGGS (SEQ ID NO: 19), GGGSGGGSGGGSGGGSGGGSGGGS (SEQ ID NO: 20), and GGGGSGGGGSGGGGSGGGGSGGGGSGGGGS (SEQ ID NO: 21).

Additionally, or alternatively, correct heavy-light chain pairing may be facilitated by swapping the CH1 and CL domains in the carboxyl-terminal Fab binding domain. By way of example, the first polypeptide, which is fused to the carboxyl terminus of the heavy chain, may comprise a VL domain and CH1 domain from the second antibody, and the second polypeptide may comprise a VH domain and CL domain from the second antibody. In another embodiment, the first polypeptide, which is fused to the carboxyl terminus of the heavy chain, may comprise a VH domain and a CL domain from the second antibody, and the second polypeptide may comprise a VL domain and CH1 domain from the second antibody.

The heavy chain constant regions or the Fc regions of the bispecific antigen binding proteins described herein may comprise one or more amino acid substitutions that affect the glycosylation and/or effector function of the antigen binding protein. One of the functions of the Fc region of an immunoglobulin is to communicate to the immune system when the immunoglobulin binds its target. This is commonly referred to as "effector function." Communication leads to antibody-dependent cellular cytotoxicity (ADCC), antibody-dependent cellular phagocytosis (ADCP), and/or complement dependent cytotoxicity (CDC). ADCC and ADCP are mediated through the binding of the Fc region to Fc receptors on the surface of cells of the immune system. CDC is mediated through the binding of the Fc with proteins of the complement system, e.g., C1q. In some embodiments, the bispecific antigen binding proteins of the invention comprise one or more amino acid substitutions in the constant region to enhance effector function, including ADCC activity, CDC activity, ADCP activity, and/or the clearance or half-life of the antigen binding protein. Exemplary amino acid substitutions (EU numbering) that can enhance effector function include, but are not limited to, E233L, L234I, L234Y, L235S, G236A, S239D, F243L, F243V, P247I, D280H, K290S, K290E, K290N, K290Y, R292P, E294L, Y296W, S298A, S298D, S298V, S298G, S298T, T299A, Y300L, V305I, Q311M, K326A, K326E, K326W, A330S, A330L, A330M, A330F, I332E, D333A, E333S, E333A, K334A, K334V, A339D, A339Q, P396L, or combinations of any of the foregoing.

In other embodiments, the bispecific antigen binding proteins of the invention comprise one or more amino acid substitutions in the constant region to reduce effector function. Exemplary amino acid substitutions (EU numbering) that can reduce effector function include, but are not limited to, C220S, C226S, C229S, E233P, L234A, L234V, V234A, L234F, L235A, L235E, G237A, P238S, S267E, H268Q, N297A, N297G, V309L, E318A, L328F, A330S, A331S, P331S or combinations of any of the foregoing.

Glycosylation can contribute to the effector function of antibodies, particularly IgG1 antibodies. Thus, in some embodiments, the bispecific antigen binding proteins of the invention may comprise one or more amino acid substitutions that affect the level or type of glycosylation of the binding proteins. Glycosylation of polypeptides is typically either N-linked or O-linked. N-linked refers to the attachment of the carbohydrate moiety to the side chain of an asparagine residue. The tri-peptide sequences asparagine-X-serine and asparagine-X-threonine, where X is any amino acid except proline, are the recognition sequences for enzymatic attachment of the carbohydrate moiety to the asparagine side chain. Thus, the presence of either of these tri-peptide sequences in a polypeptide creates a potential glycosylation site. O-linked glycosylation refers to the attachment of one of the sugars N-acetylgalactosamine, galactose, or xylose, to a hydroxyamino acid, most commonly serine or threonine, although 5-hydroxyproline or 5-hydroxylysine may also be used.

In certain embodiments, glycosylation of the bispecific antigen binding proteins described herein is increased by adding one or more glycosylation sites, e.g., to the Fc region of the binding protein. Addition of glycosylation sites to the antigen binding protein can be conveniently accomplished by altering the amino acid sequence such that it contains one or more of the above-described tri-peptide sequences (for N-linked glycosylation sites). The alteration may also be made by the addition of, or substitution by, one or more serine or threonine residues to the starting sequence (for O-linked glycosylation sites). For ease, the antigen binding protein amino acid sequence may be altered through changes at the DNA level, particularly by mutating the DNA encoding the target polypeptide at preselected bases such that codons are generated that will translate into the desired amino acids.

The invention also encompasses production of bispecific antigen binding protein molecules with altered carbohydrate structure resulting in altered effector activity, including antigen binding proteins with absent or reduced fucosylation that exhibit improved ADCC activity. Various methods are known in the art to reduce or eliminate fucosylation. For example, ADCC effector activity is mediated by binding of the antibody molecule to the FcyRIII receptor, which has been shown to be dependent on the carbohydrate structure of the N-linked glycosylation at the N297 residue of the CH2 domain. Non-fucosylated antibodies bind this receptor with increased affinity and trigger FcyRIII-mediated effector functions more efficiently than native, fucosylated antibodies. For example, recombinant production of non-fucosylated antibody in CHO cells in which the alpha-1,6-fucosyl transferase enzyme has been knocked out results in antibody with 100-fold increased ADCC activity (*see* Yamane-Ohnuki et al., Biotechnol Bioeng. 87(5):614-22, 2004). Similar effects can be accomplished through decreasing the activity of alpha-1,6-fucosyl transferase enzyme or other enzymes in the fucosylation pathway, e.g., through siRNA or antisense RNA treatment, engineering cell lines to knockout the enzyme(s), or culturing with selective glycosylation inhibitors (*see* Rothman et al., Mol Immunol. 26(12):1113-23, 1989). Some host cell strains, e.g. Lec13 or rat hybridoma YB2/0 cell line naturally produce antibodies with lower fucosylation levels (*see* Shields et al., J Biol Chem. 277(30):26733-40, 2002 and Shinkawa et al., J Biol Chem. 278(5):3466-73, 2003). An increase in the level of bisected carbohydrate, e.g. through recombinantly producing antibody in cells that overexpress GnTIII enzyme, has also been determined to increase ADCC activity (*see* Umana et al., Nat Biotechnol. 17(2):176-80, 1999).

In other embodiments, glycosylation of the bispecific antigen binding proteins described herein is decreased or eliminated by removing one or more glycosylation sites, e.g., from the Fc region of the binding protein. Amino acid substitutions that eliminate or alter N-linked glycosylation sites can reduce or eliminate N-linked glycosylation of the antigen binding protein. In certain embodiments, the bispecific antigen binding proteins described herein comprise a mutation at position N297 (EU numbering), such as N297Q, N297A, or N297G. In certain embodiments, the bispecific antigen binding proteins described herein comprise a mutation at positions L234 and L235 (EU numbering), such as L234A and L235A. In one particular embodiment, the bispecific antigen binding proteins of the invention comprise a Fc region from a human IgG1 antibody with a N297G mutation. To improve the stability of molecules comprising a N297 mutation, the Fc region of the molecules may be further engineered. For instance, in some embodiments, one or more amino acids in the Fc region are substituted with cysteine to promote disulfide bond formation in the dimeric state. Residues corresponding to V259, A287, R292, V302, L306, V323, or I332 (EU numbering) of an IgG1 Fc region may thus be substituted with cysteine. In one embodiment, specific pairs of residues are substituted with cysteine such that they preferentially form a disulfide bond with each other, thus limiting or preventing disulfide bond scrambling. In certain embodiments pairs include, but are not limited to, A287C and L306C, V259C and L306C, R292C and V302C, and V323C and I332C. In particular embodiments, the bispecific antigen binding proteins described herein comprise a Fc region from a human IgG1 antibody with mutations at R292C and V302C. In such embodiments, the Fc region may also comprise a N297G mutation.

Modifications of the bispecific antigen binding proteins of the invention to increase serum half-life also may desirable, for example, by incorporation of or addition of a salvage receptor binding epitope (e.g., by mutation of the appropriate region or by incorporating the epitope into a peptide tag that is then fused to the antigen binding protein at either end or in the middle, e.g., by DNA or peptide synthesis; *see, e.g.,* WO96/32478) or adding molecules such as PEG or other water soluble polymers, including polysaccharide polymers. The salvage receptor binding epitope preferably constitutes a region wherein any one or more amino acid residues from one or two loops of a Fc region are transferred to an analogous position in the antigen binding protein. In one embodiment, three or more residues from one or two loops of the Fc region are transferred. In one embodiment, the epitope is taken from the CH2 domain of the Fc region (e.g., an IgG Fc region) and transferred to the CH1, CH3, or VH region, or more than one such region, of the antigen binding protein. Alternatively, the epitope is taken from the CH2 domain of the Fc region and transferred to the CL region or VL region, or both, of the antigen binding protein. *See* International applications WO 97/34631 and WO 96/32478 for a description of Fc variants and their interaction with the salvage receptor.

The present invention includes one or more isolated nucleic acids encoding the bispecific antigen binding proteins and components thereof described herein. Nucleic acid molecules of the invention include DNA and RNA in both single-stranded and double-stranded form, as well as the corresponding complementary sequences. DNA includes, for example, cDNA, genomic DNA, chemically synthesized DNA, DNA amplified by PCR, and combinations thereof. The nucleic acid molecules of the invention include full-length genes or cDNA molecules as well as a combination of fragments thereof. In one embodiment, the nucleic acids of the invention are derived from human sources, but the invention includes those derived from non-human species, as well.

Relevant amino acid sequences from an immunoglobulin or region thereof (e.g. variable region, Fc region, etc.) or polypeptide of interest may be determined by direct protein sequencing, and suitable encoding nucleotide sequences can be designed according to a universal codon table. Alternatively, genomic or cDNA encoding monoclonal antibodies from which the binding domains of the bispecific antigen binding proteins of the invention may be derived can be isolated and sequenced from cells producing such antibodies using conventional procedures (e.g., by using oligonucleotide probes that are capable of binding specifically to genes encoding the heavy and light chains of the monoclonal antibodies).

An "isolated nucleic acid," which is used interchangeably herein with "isolated polynucleotide," is a nucleic acid that has been separated from adjacent genetic sequences present in the genome of the organism from which the nucleic acid was isolated, in the case of nucleic acids isolated from naturally- occurring sources. In the case of nucleic acids synthesized enzymatically from a template or chemically, such as PCR products, cDNA molecules, or oligonucleotides for example, it is understood that the nucleic acids resulting from such processes are isolated nucleic acids. An isolated nucleic acid molecule refers to a nucleic acid molecule in the form of a separate fragment or as a component of a larger nucleic acid construct. In one embodiment, the nucleic acids are substantially free from contaminating endogenous material. The nucleic acid molecule has been derived from DNA or RNA isolated at least once in substantially pure form and in a quantity or concentration enabling identification, manipulation, and recovery of its component nucleotide sequences by standard biochemical methods (such as those outlined in Sambrook et al., Molecular Cloning: A Laboratory Manual, 2nd ed., Cold Spring Harbor Laboratory, Cold Spring Harbor, NY

(1989)). Such sequences are provided and/or constructed in the form of an open reading frame uninterrupted by internal non-translated sequences, or introns, that are typically present in eukaryotic genes. Sequences of non-translated DNA can be present 5' or 3' from an open reading frame, where the same do not interfere with manipulation or expression of the coding region. Unless specified otherwise, the left-hand end of any single-stranded polynucleotide sequence discussed herein is the 5' end; the left-hand direction of double-stranded polynucleotide sequences is referred to as the 5' direction. The direction of 5' to 3' production of nascent RNA transcripts is referred to as the transcription direction; sequence regions on the DNA strand having the same sequence as the RNA transcript that are 5' to the 5' end of the RNA transcript are referred to as "upstream sequences;" sequence regions on the DNA strand having the same sequence as the RNA transcript that are 3' to the 3' end of the RNA transcript are referred to as "downstream sequences."

Variants of the antigen binding proteins described herein can be prepared by site-specific mutagenesis of nucleotides in the DNA encoding the polypeptide, using cassette or PCR mutagenesis or other techniques well known in the art, to produce DNA encoding the variant, and thereafter expressing the recombinant DNA in cell culture as outlined herein. However, antigen binding proteins comprising variant CDRs having up to about 100-150 residues may be prepared by *in vitro* synthesis using established techniques. The variants typically exhibit the same qualitative biological activity as the naturally occurring analogue, e.g., binding to antigen. Such variants include, for example, deletions and/or insertions and/or substitutions of residues within the amino acid sequences of the antigen binding proteins. Any combination of deletion, insertion, and substitution is made to arrive at the final construct, provided that the final construct possesses the desired characteristics. The amino acid changes also may alter post-translational processes of the antigen binding protein, such as changing the number or position of glycosylation sites. In certain embodiments, antigen binding protein variants are prepared with the intent to modify those amino acid residues which are directly involved in epitope binding. In other embodiments, modification of residues which are not directly involved in epitope binding or residues not involved in epitope binding in any way, is desirable, for purposes discussed herein. Mutagenesis within any of the CDR regions and/or framework regions is contemplated. Covariance analysis techniques can be employed by the skilled artisan to design useful modifications in the amino acid sequence of the antigen binding protein. *See, e.g.,* Choulier, et al., Proteins 41:475-484, 2000; Demarest et al., J. Mol. Biol. 335:41-48, 2004; Hugo et al., Protein Engineering 16(5):381-86, 2003; Aurora et al., US Patent Publication No. 2008/0318207 A1; Glaser et al., US Patent Publication No. 2009/0048122 A1; Urech et al., WO 2008/110348 A1; Borras et al., WO 2009/000099 A2. Such modifications determined by covariance analysis can improve potency, pharmacokinetic, pharmacodynamic, and/or manufacturability characteristics of an antigen binding protein.

The nucleic acid sequences of the present invention. As will be appreciated by those in the art, due to the degeneracy of the genetic code, an extremely large number of nucleic acids may be made, all of which encode the CDRs (and heavy and light chains or other components of the antigen binding proteins described herein) of the invention. Thus, having identified a particular amino acid sequence, those skilled in the art could make any number of different nucleic acids, by simply modifying the sequence of one or more codons in a way which does not change the amino acid sequence of the encoded protein.

The present invention also includes vectors comprising one or more nucleic acids encoding one or more components of the bispecific antigen binding proteins of the invention (e.g. variable regions, light chains, heavy chains, modified heavy chains, and Fd fragments). The term "vector" refers to any molecule or entity (e.g., nucleic acid, plasmid, bacteriophage or virus) used to transfer protein coding information into a host cell. Examples of vectors include, but are not limited to, plasmids, viral vectors, non-episomal mammalian vectors and expression vectors, for example, recombinant expression vectors. The term "expression vector" or "expression construct" as used herein refers to a recombinant DNA molecule containing a desired coding sequence and appropriate nucleic acid control sequences necessary for the expression of the operably linked coding sequence in a particular host cell. An expression vector can include, but is not limited to, sequences that affect or control transcription, translation, and, if introns are present, affect RNA splicing of a coding region operably linked thereto. Nucleic acid sequences necessary for expression in prokaryotes include a promoter, optionally an operator sequence, a ribosome binding site and possibly other sequences. Eukaryotic cells are known to utilize promoters, enhancers, and termination and polyadenylation signals. A secretory signal peptide sequence can also, optionally, be encoded by the expression vector, operably linked to the coding sequence of interest, so that the expressed polypeptide can be secreted by the recombinant host cell, for more facile isolation of the polypeptide of interest from the cell, if desired. For instance, in some embodiments, signal peptide sequences may be appended/fused to the amino terminus of any of the polypeptides sequences of the present invention. In certain embodiments, a signal peptide having the amino acid sequence of MDMRVPAQLLGLLLLWLRGARC (SEQ ID NO: 22) is fused to the amino terminus of any of the polypeptide sequences of the present invention. In other embodiments, a signal peptide having the amino acid sequence of MAWALLLLTLLTQGTGSWA (SEQ ID NO: 23) is fused to the amino terminus of any of the polypeptide sequences of the present invention. In still other embodiments, a signal peptide having the amino acid sequence of MTCSPLLLTLLIHCTGSWA (SEQ ID NO: 24) is fused to the amino terminus of any of the polypeptide sequences of the present invention. Other suitable signal peptide sequences that can be fused to the amino terminus of the polypeptide sequences described herein include: MEAPAQLLFLLLLWLPDTTG (SEQ ID NO: 25), MEWTWRVLFLVAAATGAHS (SEQ ID NO: 26), METPAQLLFLLLLWLPDTTG (SEQ ID NO: 27), METPAQLLFLLLLWLPDTTG (SEQ ID NO: 28), MKHLWFFLLLVAAPRWVLS (SEQ ID NO: 29), and MEWSWVFLFFLSVTTGVHS (SEQ ID NO: 30). Other signal peptides are known to those of skill in the art and may be fused to any of the polypeptide chains of the present invention, for example, to facilitate or optimize expression in particular host cells.

Typically, expression vectors used in the host cells to produce the bispecific antigen proteins of the invention will contain sequences for plasmid maintenance and for cloning and expression of exogenous nucleotide sequences encoding the components of the bispecific antigen binding proteins. Such sequences, collectively referred to as "flanking sequences," in certain embodiments will typically include one or more of the following nucleotide sequences: a promoter, one or more enhancer sequences, an origin of replication, a transcriptional termination sequence, a complete intron sequence containing a donor and acceptor splice site, a sequence encoding a leader sequence for polypeptide secretion, a ribosome binding site, a polyadenylation sequence, a polylinker region for inserting the nucleic acid encoding the polypeptide to be expressed, and a selectable marker element. Each of these sequences is discussed below.

Optionally, the vector may contain a "tag"-encoding sequence, i.e., an oligonucleotide molecule located at the 5' or 3' end of the polypeptide coding sequence; the oligonucleotide tag sequence encodes polyHis (such as hexaHis), FLAG, HA (hemaglutinin influenza virus), myc, or another "tag" molecule for which commercially available antibodies exist. This tag is typically fused to the polypeptide upon expression of the polypeptide, and can serve as a means for affinity purification or detection of the polypeptide from the host cell. Affinity purification can be accomplished, for example, by column chromatography using antibodies against the tag as an affinity matrix. Optionally, the tag can subsequently be removed from the purified polypeptide by various means such as using certain peptidases for cleavage.

Flanking sequences may be homologous (i.e., from the same species and/or strain as the host cell), heterologous (i.e., from a species other than the host cell species or strain), hybrid (i.e., a combination of flanking sequences from more than one source), synthetic or native. As such, the source of a flanking sequence may be any prokaryotic or eukaryotic organism, any vertebrate or invertebrate organism, or any plant, provided that the flanking sequence is functional in, and can be activated by, the host cell machinery.

Flanking sequences useful in the vectors of this invention may be obtained by any of several methods well known in the art. Typically, flanking sequences useful herein will have been previously identified by mapping and/or by restriction endonuclease digestion and can thus be isolated from the proper tissue source using the appropriate restriction endonucleases. In some cases, the full nucleotide sequence of a flanking sequence may be known. Here, the flanking sequence may be synthesized using routine methods for nucleic acid synthesis or cloning.

Whether all or only a portion of the flanking sequence is known, it may be obtained using polymerase chain reaction (PCR) and/or by screening a genomic library with a suitable probe such as an oligonucleotide and/or flanking sequence fragment from the same or another species. Where the flanking sequence is not known, a fragment of DNA containing a flanking sequence may be isolated from a larger piece of DNA that may contain, for example, a coding sequence or even another gene or genes. Isolation may be accomplished by restriction endonuclease digestion to produce the proper DNA fragment followed by isolation using agarose gel purification, Qiagen^{®} column chromatography (Chatsworth, CA), or other methods known to the skilled artisan. The selection of suitable enzymes to accomplish this purpose will be readily apparent to one of ordinary skill in the art.

An origin of replication is typically a part of those prokaryotic expression vectors purchased commercially, and the origin aids in the amplification of the vector in a host cell. If the vector of choice does not contain an origin of replication site, one may be chemically synthesized based on a known sequence, and ligated into the vector. For example, the origin of replication from the plasmid pBR322 (New England Biolabs, Beverly, MA) is suitable for most gram-negative bacteria, and various viral origins (e.g., SV40, polyoma, adenovirus, vesicular stomatitus virus (VSV), or papillomaviruses such as HPV or BPV) are useful for cloning vectors in mammalian cells. Generally, the origin of replication component is not needed for mammalian expression vectors (for example, the SV40 origin is often used only because it also contains the virus early promoter).

A transcription termination sequence is typically located 3' to the end of a polypeptide coding region and serves to terminate transcription. Usually, a transcription termination sequence in prokaryotic cells is a G-C rich fragment followed by a poly-T sequence. While the sequence is easily cloned from a library or even purchased commercially as part of a vector, it can also be readily synthesized using known methods for nucleic acid synthesis.

A selectable marker gene encodes a protein necessary for the survival and growth of a host cell grown in a selective culture medium. Typical selection marker genes encode proteins that (a) confer resistance to antibiotics or other toxins, e.g., ampicillin, tetracycline, or kanamycin for prokaryotic host cells; (b) complement auxotrophic deficiencies of the cell; or (c) supply critical nutrients not available from complex or defined media. Specific selectable markers are the kanamycin resistance gene, the ampicillin resistance gene, and the tetracycline resistance gene. Advantageously, a neomycin resistance gene may also be used for selection in both prokaryotic and eukaryotic host cells.

Other selectable genes may be used to amplify the gene that will be expressed. Amplification is the process wherein genes that are required for production of a protein critical for growth or cell survival are reiterated in tandem within the chromosomes of successive generations of recombinant cells. Examples of suitable selectable markers for mammalian cells include dihydrofolate reductase (DHFR) and promoterless thymidine kinase genes. Mammalian cell transformants are placed under selection pressure wherein only the transformants are uniquely adapted to survive by virtue of the selectable gene present in the vector. Selection pressure is imposed by culturing the transformed cells under conditions in which the concentration of selection agent in the medium is successively increased, thereby leading to the amplification of both the selectable gene and the DNA that encodes another gene, such as one or more components of the bispecific antigen binding proteins described herein. As a result, increased quantities of a polypeptide are synthesized from the amplified DNA.

A ribosome-binding site is usually necessary for translation initiation of mRNA and is characterized by a Shine-Dalgarno sequence (prokaryotes) or a Kozak sequence (eukaryotes). The element is typically located 3' to the promoter and 5' to the coding sequence of the polypeptide to be expressed. In certain embodiments, one or more coding regions may be operably linked to an internal ribosome binding site (IRES), allowing translation of two open reading frames from a single RNA transcript.

In some cases, such as where glycosylation is desired in a eukaryotic host cell expression system, one may manipulate the various pre- or prosequences to improve glycosylation or yield. For example, one may alter the peptidase cleavage site of a particular signal peptide, or add prosequences, which also may affect glycosylation. The final protein product may have, in the -1 position (relative to the first amino acid of the mature protein) one or more additional amino acids incident to expression, which may not have been totally removed. For example, the final protein product may have one or two amino acid residues found in the peptidase cleavage site, attached to the amino-terminus. Alternatively, use of some enzyme cleavage sites may result in a slightly truncated form of the desired polypeptide, if the enzyme cuts at such area within the mature polypeptide.

Expression and cloning vectors of the invention will typically contain a promoter that is recognized by the host organism and operably linked to the molecule encoding the polypeptide. The term "operably linked" as used herein refers to the linkage of two or more nucleic acid sequences in such a manner that a nucleic acid molecule capable of directing the transcription of a given gene and/or the synthesis of a desired protein molecule is produced. For example, a control sequence in a vector that is "operably linked" to a protein coding sequence is ligated thereto so that expression of the protein coding sequence is achieved under conditions compatible with the transcriptional activity of the control sequences. More specifically, a promoter and/or enhancer sequence, including any combination of cis-acting transcriptional control elements is operably linked to a coding sequence if it stimulates or modulates the transcription of the coding sequence in an appropriate host cell or other expression system.

Promoters are untranscribed sequences located upstream (i.e., 5') to the start codon of a structural gene (generally within about 100 to 1000 bp) that control transcription of the structural gene. Promoters are conventionally grouped into one of two classes: inducible promoters and constitutive promoters. Inducible promoters initiate increased levels of transcription from DNA under their control in response to some change in culture conditions, such as the presence or absence of a nutrient or a change in temperature. Constitutive promoters, on the other hand, uniformly transcribe a gene to which they are operably linked, that is, with little or no control over gene expression. A large number of promoters, recognized by a variety of potential host cells, are well known. A suitable promoter is operably linked to the DNA encoding e.g., heavy chain, light chain, modified heavy chain, or other component of the bispecific antigen binding proteins of the invention, by removing the promoter from the source DNA by restriction enzyme digestion and inserting the desired promoter sequence into the vector.

Suitable promoters for use with yeast hosts are also well known in the art. Yeast enhancers are advantageously used with yeast promoters. Suitable promoters for use with mammalian host cells are well known and include, but are not limited to, those obtained from the genomes of viruses such as polyoma virus, fowlpox virus, adenovirus (such as Adenovirus 2), bovine papilloma virus, avian sarcoma virus, cytomegalovirus, retroviruses, hepatitis-B virus and Simian Virus 40 (SV40). Other suitable mammalian promoters include heterologous mammalian promoters, for example, heat-shock promoters and the actin promoter.

Additional promoters which may be of interest include, but are not limited to: SV40 early promoter (Benoist and Chambon, 1981, Nature 290:304-310); CMV promoter (Thornsen et al., 1984, Proc. Natl. Acad. U.S.A. 81:659-663); the promoter contained in the 3' long terminal repeat of Rous sarcoma virus (Yamamoto et al., 1980, Cell 22:787-797); herpes thymidine kinase promoter (Wagner et al., 1981, Proc. Natl. Acad. Sci. U.S.A. 78: 1444-1445); promoter and regulatory sequences from the metallothionine gene Prinster et al., 1982, Nature 296:39-42); and prokaryotic promoters such as the beta-lactamase promoter (Villa-Kamaroff et al., 1978, Proc. Natl. Acad. Sci. U.S.A. 75:3727-3731); or the tac promoter (DeBoer et al., 1983, Proc. Natl. Acad. Sci. U.S.A. 80:21-25). Also of interest are the following animal transcriptional control regions, which exhibit tissue specificity and have been utilized in transgenic animals: the elastase I gene control region that is active in pancreatic acinar cells (Swift et al., 1984, Cell 38:639-646; Omitz et al., 1986, Cold Spring Harbor Symp. Quant. Biol. 50:399-409; MacDonald, 1987, Hepatology 7:425-515); the insulin gene control region that is active in pancreatic beta cells (Hanahan, 1985, Nature 315: 115-122); the immunoglobulin gene control region that is active in lymphoid cells (Grosschedl et al., 1984, Cell 38:647-658; Adames et al., 1985, Nature 318:533-538; Alexander et al., 1987, Mol. Cell. Biol. 7: 1436-1444); the mouse mammary tumor virus control region that is active in testicular, breast, lymphoid and mast cells (Leder et al., 1986, Cell 45:485-495); the albumin gene control region that is active in liver (Pinkert et al., 1987, Genes and Devel. 1 :268-276); the alpha-feto-protein gene control region that is active in liver (Krumlauf et al., 1985, Mol. Cell. Biol. 5: 1639-1648; Hammer et al., 1987, Science 253:53-58); the alpha 1-antitrypsin gene control region that is active in liver (Kelsey et al., 1987, Genes and Devel. 1: 161-171); the beta-globin gene control region that is active in myeloid cells (Mogram et al, 1985, Nature 315:338-340; Kollias et al, 1986, Cell 46:89-94); the myelin basic protein gene control region that is active in oligodendrocyte cells in the brain (Readhead et al., 1987, Cell 48:703-712); the myosin light chain-2 gene control region that is active in skeletal muscle (Sani, 1985, Nature 314:283-286); and the gonadotropic releasing hormone gene control region that is active in the hypothalamus (Mason et al., 1986, Science 234: 1372-1378).

An enhancer sequence may be inserted into the vector to increase transcription of DNA encoding a component of the bispecific antigen binding proteins (e.g., light chain, heavy chain, modified heavy chain, Fd fragment) by higher eukaryotes. Enhancers are cis-acting elements of DNA, usually about 10-300 bp in length, that act on the promoter to increase transcription. Enhancers are relatively orientation and position independent, having been found at positions both 5' and 3' to the transcription unit. Several enhancer sequences available from mammalian genes are known (e.g., globin, elastase, albumin, alpha-feto-protein and insulin). Typically, however, an enhancer from a virus is used. The SV40 enhancer, the cytomegalovirus early promoter enhancer, the polyoma enhancer, and adenovirus enhancers known in the art are exemplary enhancing elements for the activation of eukaryotic promoters. While an enhancer may be positioned in the vector either 5' or 3' to a coding sequence, it is typically located at a site 5' from the promoter. A sequence encoding an appropriate native or heterologous signal sequence (leader sequence or signal peptide) can be incorporated into an expression vector, to promote extracellular secretion of the antibody. The choice of signal peptide or leader depends on the type of host cells in which the antibody is to be produced, and a heterologous signal sequence can replace the native signal sequence. Examples of signal peptides are described above. Other signal peptides that are functional in mammalian host cells include the signal sequence for interleukin-7 (IL-7) described in US Patent No. 4,965,195; the signal sequence for interleukin-2 receptor described in Cosman et al.,1984, Nature 312:768; the interleukin-4 receptor signal peptide described in EP Patent No. 0367 566; the type I interleukin-1 receptor signal peptide described in U.S. Patent No. 4,968,607; the type II interleukin-1 receptor signal peptide described in EP Patent No. 0 460 846.

The expression vectors that are provided may be constructed from a starting vector such as a commercially available vector. Such vectors may or may not contain all of the desired flanking sequences. Where one or more of the flanking sequences described herein are not already present in the vector, they may be individually obtained and ligated into the vector. Methods used for obtaining each of the flanking sequences are well known to one skilled in the art. The expression vectors can be introduced into host cells to thereby produce proteins, including fusion proteins, encoded by nucleic acids as described herein.

In certain embodiments, nucleic acids encoding the different components of the bispecific antigen binding proteins of the invention may be inserted into the same expression vector. For instance, the nucleic acid encoding an anti-first target antigen light chain can be cloned into the same vector as the nucleic acid encoding an anti- first target antigen heavy chain. In such embodiments, the two nucleic acids may be separated by an internal ribosome entry site (IRES) and under the control of a single promoter such that the light chain and heavy chain are expressed from the same mRNA transcript. Alternatively, the two nucleic acids may be under the control of two separate promoters such that the light chain and heavy chain are expressed from two separate mRNA transcripts. In some embodiments, nucleic acids encoding the anti- first target antigen light chain and heavy chain are cloned into one expression vector and the nucleic acids encoding the anti- second target antigen light chain and heavy chain are cloned into a second expression vector.

Similarly, for IgG-Fab bispecific antigen binding proteins, nucleic acids encoding each of the three components may be cloned into the same expression vector. In some embodiments, the nucleic acid encoding the light chain of the IgG-Fab molecule and the nucleic acid encoding the second polypeptide (which comprises the other half of the C-terminal Fab domain) are cloned into one expression vector, whereas the nucleic acid encoding the modified heavy chain (fusion protein comprising a heavy chain and half of a Fab domain) is cloned into a second expression vector. In certain embodiments, all components of the bispecific antigen binding proteins described herein are expressed from the same host cell population. For example, even if one or more components is cloned into a separate expression vector, the host cell is co-transfected with both expression vectors such that one cell produces all components of the bispecific antigen binding proteins.

After the vector has been constructed and the one or more nucleic acid molecules encoding the components of the bispecific antigen binding proteins described herein has been inserted into the proper site(s) of the vector or vectors, the completed vector(s) may be inserted into a suitable host cell for amplification and/or polypeptide expression. Thus, the present invention encompasses an isolated host cell comprising one or more expression vectors encoding the components of the bispecific antigen binding proteins. The term "host cell" as used herein refers to a cell that has been transformed, or is capable of being transformed, with a nucleic acid and thereby expresses a gene of interest. The term includes the progeny of the parent cell, whether or not the progeny is identical in morphology or in genetic make-up to the original parent cell, so long as the gene of interest is present. A host cell that comprises an isolated nucleic acid of the invention, in one embodiment operably linked to at least one expression control sequence (e.g. promoter or enhancer), is a "recombinant host cell."

The transformation of an expression vector for an antigen binding protein into a selected host cell may be accomplished by well-known methods including transfection, infection, calcium phosphate co-precipitation, electroporation, microinjection, lipofection, DEAE-dextran mediated transfection, or other known techniques. The method selected will in part be a function of the type of host cell to be used. These methods and other suitable methods are well known to the skilled artisan, and are set forth, for example, in Sambrook et al., 2001, *supra.*

A host cell, when cultured under appropriate conditions, synthesizes an antigen binding protein that can subsequently be collected from the culture medium (if the host cell secretes it into the medium) or directly from the host cell producing it (if it is not secreted). The selection of an appropriate host cell will depend upon various factors, such as desired expression levels, polypeptide modifications that are desirable or necessary for activity (such as glycosylation or phosphorylation) and ease of folding into a biologically active molecule.

Exemplary host cells include prokaryote, yeast, or higher eukaryote cells. Prokaryotic host cells include eubacteria, such as Gram-negative or Gram-positive organisms, for example, *Enterobacteriaceae* such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacillus,* such as *B. subtilis* and *B. licheniformis, Pseudomonas,* and *Streptomyces.* Eukaryotic microbes such as filamentous fungi or yeast are suitable cloning or expression hosts for recombinant polypeptides. *Saccharomyces cerevisiae,* or common baker's yeast, is the most commonly used among lower eukaryotic host microorganisms. However, a number of other genera, species, and strains are commonly available and useful herein, such as *Pichia,* e.g. *P. pastoris, Schizosaccharomyces pombe; Kluyveromyces, Yarrowia; Candida; Trichoderma reesia; Neurospora crassa; Schwanniomyces,* such as *Schwanniomyces occidentalis;* and filamentous fungi, such as, e.g., *Neurospora, Penicillium, Tolypocladium, and Aspergillus* hosts such *as A. nidulans and A. niger.*

Host cells for the expression of glycosylated antigen binding proteins can be derived from multicellular organisms. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified. A variety of viral strains for transfection of such cells are publicly available, e.g., the L-1 variant *of Autographa californica* NPV and the Bm-5 strain of *Bombyx mori* NPV.

Vertebrate host cells are also suitable hosts, and recombinant production of antigen binding proteins from such cells has become routine procedure. Mammalian cell lines available as hosts for expression are well known in the art and include, but are not limited to, immortalized cell lines available from the American Type Culture Collection (ATCC), including but not limited to Chinese hamster ovary (CHO) cells, including CHOK1 cells (ATCC CCL61), DXB-11, DG-44, and Chinese hamster ovary cells/-DHFR (CHO, Urlaub et al., Proc. Natl. Acad. Sci. USA 77: 4216, 1980); monkey kidney CV1 line transformed by SV40 (COS-7, ATCC CRL 1651); human embryonic kidney line (293 or 293 cells subcloned for growth in suspension culture, (Graham et al., J. Gen Virol. 36: 59, 1977); baby hamster kidney cells (BHK, ATCC CCL 10); mouse sertoli cells (TM4, Mather, Biol. Reprod. 23: 243-251, 1980); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human hepatoma cells (Hep G2, HB 8065); mouse mammary tumor (MMT 060562, ATCC CCL51); TRI cells (Mather et al., Annals N.Y Acad. Sci. 383: 44-68, 1982); MRC 5 cells or FS4 cells; mammalian myeloma cells, and a number of other cell lines. In certain embodiments, cell lines may be selected through determining which cell lines have high expression levels and constitutively produce bispecific antigen binding proteins of the present invention. In another embodiment, a cell line from the B cell lineage that does not make its own antibody but has a capacity to make and secrete a heterologous antibody can be selected. CHO cells are host cells in some embodiments for expressing the bispecific antigen binding proteins of the invention.

Host cells are transformed or transfected with the above-described nucleic acids or vectors for production of bispecific antigen binding proteins and are cultured in conventional nutrient media modified as appropriate for inducing promoters, selecting transformants, or amplifying the genes encoding the desired sequences. In addition, novel vectors and transfected cell lines with multiple copies of transcription units separated by a selective marker are particularly useful for the expression of antigen binding proteins. Thus, the present invention also provides a method for preparing a bispecific antigen binding protein described herein comprising culturing a host cell comprising one or more expression vectors described herein in a culture medium under conditions permitting expression of the bispecific antigen binding protein encoded by the one or more expression vectors; and recovering the bispecific antigen binding protein from the culture medium.

The host cells used to produce the antigen binding proteins of the invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58: 44, 1979; Barnes et al., Anal. Biochem. 102: 255, 1980; U.S. Patent Nos. 4,767,704; 4,657,866; 4,927,762; 4,560,655; or 5,122,469; WO90103430; WO 87/00195; or U.S. Patent Re. No. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as Gentamycin^{™} drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

Upon culturing the host cells, the bispecific antigen binding protein can be produced intracellularly, in the periplasmic space, or directly secreted into the medium. If the antigen binding protein is produced intracellularly, as a first step, the particulate debris, either host cells or lysed fragments, is removed, for example, by centrifugation or ultrafiltration. The bispecific antigen binding protein can be purified using, for example, hydroxyapatite chromatography, cation or anion exchange chromatography, or affinity chromatography, using the antigen(s) of interest or protein A or protein G as an affinity ligand. Protein A can be used to purify proteins that include polypeptides that are based on human γ1, *y2,* or γ4 heavy chains (Lindmark et al., J. Immunol. Meth. 62: 1-13, 1983). Protein G is recommended for all mouse isotypes and for human γ3 (Guss et al., EMBO J. 5: 15671575, 1986). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the protein comprises a CH3 domain, the Bakerbond ABX^{™} resin (J. T. Baker, Phillipsburg, N.J.) is useful for purification. Other techniques for protein purification such as ethanol precipitation, Reverse Phase HPLC, chromatofocusing, SDS-PAGE, and ammonium sulfate precipitation are also possible depending on the particular bispecific antigen binding protein to be recovered.

The bispecific antigen binding proteins of the invention are useful for detecting target antigen(s) in biological samples and identification of cells or tissues that express the target antigen(s). The bispecific antigen binding proteins described herein can be used for diagnostic purposes to detect, diagnose, or monitor diseases and/or conditions associated with the target antigen(s). Also provided are methods for the detection of the presence of the target antigen(s) in a sample using classical immunohistological methods known to those of skill in the art (e.g., Tijssen, 1993, Practice and Theory of Enzyme Immunoassays, Vol 15 (Eds R.H. Burdon and P.H. van Knippenberg, Elsevier, Amsterdam); Zola, 1987, Monoclonal Antibodies: A Manual of Techniques, pp. 147-158 (CRC Press, Inc.); Jalkanen et al., 1985, J. Cell. Biol. 101:976-985; Jalkanen et al., 1987, J. Cell Biol. 105:3087-3096). The detection of either target can be performed *in vivo* or *in vitro.*

Diagnostic applications provided herein include use of the antigen binding proteins to detect expression of target antigen(s). Examples of methods useful in the detection of the presence of the receptor include immunoassays, such as the enzyme linked immunosorbent assay (ELISA) and the radioimmunoassay (RIA).

For diagnostic applications, the antigen binding protein typically will be labeled with a detectable labeling group. Suitable labeling groups include, but are not limited to, the following: radioisotopes or radionuclides (e.g., ³H, ¹⁴C, ¹⁵N, ³⁵S, ⁹⁰Y, ⁹⁹Tc, ¹¹¹In, ¹²⁵I, ¹³¹I), fluorescent groups (e.g., FITC, rhodamine, lanthanide phosphors), enzymatic groups (e.g., horseradish peroxidase, β-galactosidase, luciferase, alkaline phosphatase), chemiluminescent groups, biotinyl groups, or predetermined polypeptide epitopes recognized by a secondary reporter (e.g., leucine zipper pair sequences, binding sites for secondary antibodies, metal binding domains, epitope tags). In some embodiments, the labeling group is coupled to the antigen binding protein via spacer arms of various lengths to reduce potential steric hindrance. Various methods for labeling proteins are known in the art and may be used.

In another embodiment, the bispecific antigen binding protein described herein can be used to identify a cell or cells that express target antigen(s). In a specific embodiment, the antigen binding protein is labeled with a labeling group and the binding of the labeled antigen binding protein to target antigen(s) is detected. In a further specific embodiment, the binding of the antigen binding protein to target antigen(s) is detected *in vivo.* In a further specific embodiment, the bispecific antigen binding protein is isolated and measured using techniques known in the art. *See,* for example, Harlow and Lane, 1988, Antibodies: A Laboratory Manual, New York: Cold Spring Harbor (ed. 1991 and periodic supplements); John E. Coligan, ed., 1993, Current Protocols In Immunology New York: John Wiley & Sons.

### Examples

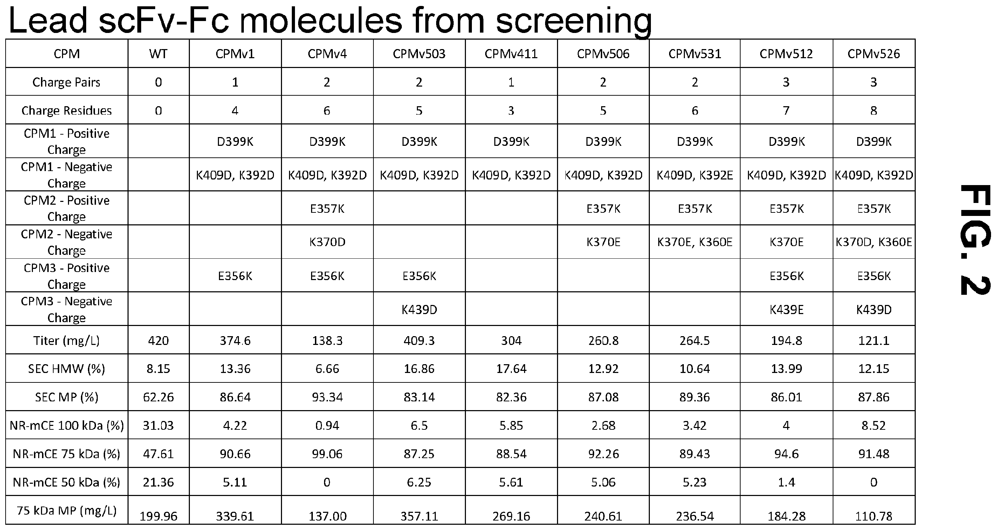

Based on crystal structures of monoclonal antibodies with and without modified salt bridges, K360 has been identified as an interface residue between two Fc chains. Early experiments that were done to screen for improved pairing and structure of Hetero-Fc molecules were done using the combination of a single chain variable fraction (scFv) fused to an Fc with an Fc chain lacking a warhead. When assembled with the intended hetero-Fc format, the molecular weight was approximately 75 kilo Daltons (kDa), as shown in Figure 1. In contrast, when the molecules assembled into homodimers with two scFv warheads or lacking warheads all together, the molecular weights were approximately 100 kDa or 50 kDa respectively. In the production of these molecules, modified fragments were synthesized and cloned into plasmids using the golden gate cloning strategy. The resulting panel of recombinant molecules was generated using transient expression of HEK 293 EBNA1 cells in suspension in accordance with methods established and published by NRCC. After six days of production, cultured media (CM) was harvested from the cell cultures and proceeded to protein A purification in accordance with common established practices, followed by analysis. In the analysis, ratios of non-covalent aggregation and desired species were measured using size exclusion chromatography (SEC) according to industry established methods. Ratios of homo-dimer and hetero-dimer formation were measured using a Caliper SDS micro-capillary gel electrophoresis (mCE) instrument based on travel due primarily to molecular size. From the initial screen of 44 variants, a set of molecules with favorable pairing specificity and expression titer were selected for further optimization and analysis. Measurements of the lead variants in the screen are shown in Figure 2. It is evident that each of the variants selected has improved pairing specificity when compared with WT (47.61% hetero-scFv-Fc) with levels ranging from 89.4% to 94.6%. The amount of hetero-scFv-Fc being produced was calculated from the production titer and percent main peak as calculated by mCE and shown under 75 kDa MP (mg/L) in Figure 2. By those measurements, many variants had more desired species than WT and some had more desired species than the established variant CPMv1.

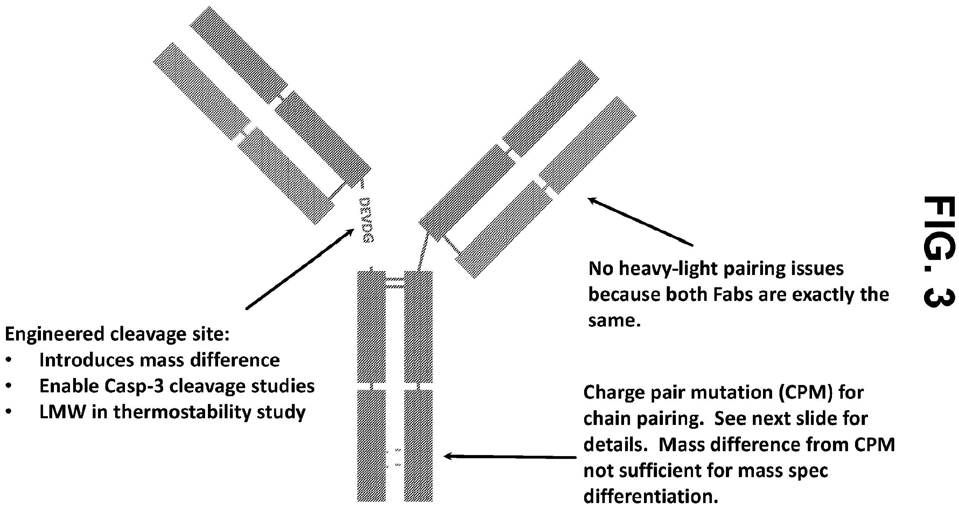

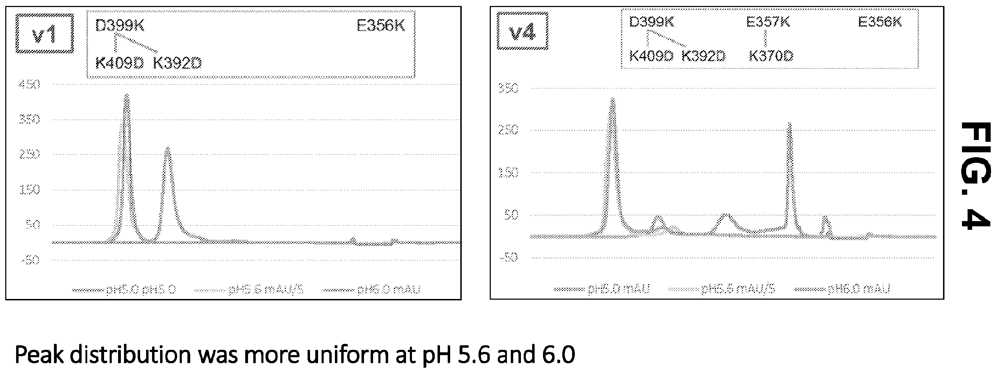

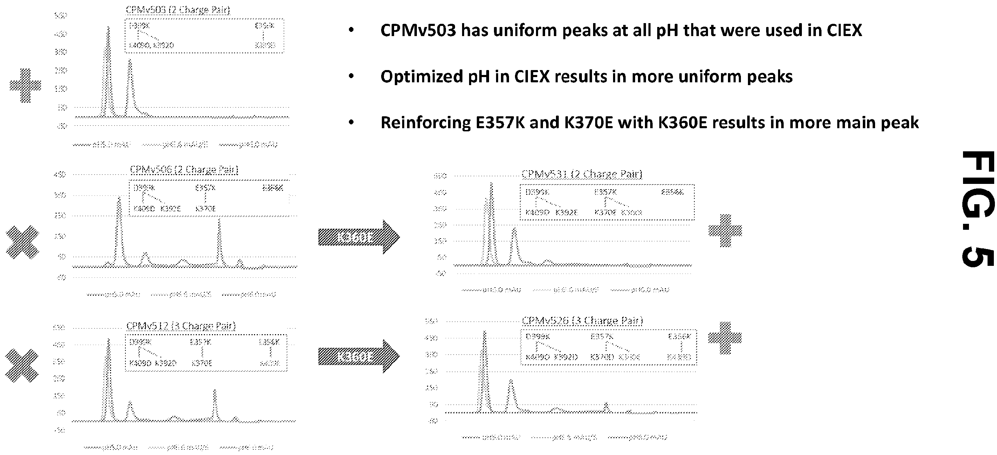

In the second experiment, the lead variants were tested in a modified monoclonal antibody. Modifications include the addition of charge pair mutations in the CH3 region and the addition of a DEVD cleavage site in the hinge between one of the Fab and Fc regions, as shown in Figure 3. This format was used instead of a hetero-IgG to eliminate variability introduced by non-specific pairing between the light chain and heavy chain. In this experiment, productions were done using a CHO-K1 stable expression system coupled with internal vectors. Titers from those productions are in the range of 186 mg/L to 275 mg/L, showing fairly low variance. Purification began with protein A capture. That was followed with the removal of HMW species by SEC according to established practices. The process was then completed with isolation from monoclonal antibodies by cation exchange (CEX) in accordance with industry standard practices. Fractions were selected for pooling according to purity of desired product as measured by SEC and MS. During the CEX stage of purification, some variants were observed to split into multiple peaks. When these peaks were measured by MS, they were predominantly species of matching molecular weight. In addition, when samples from one peak were collected and re-loaded into CEX, they separated into the same pattern of multiple peaks observed before (data not shown). This indicates that those variants had a degree of instability that led to conformational changes when exposed to the specific conditions of the CEX column. This phenomenon was especially prominent when the buffers used for CEX were at pH 5.0, as shown in Figure 4, while the calculated pI of the molecules was in the range of 7.93. Peak distribution was more uniform when buffers were at pH 5.6 and 6.0. It was also observed that the formation of multiple peaks took place when the CPM at E357K with K370D/E was used in combination with the CPM at D399K with K409D and K392D/E. When the charge pair at E357K with K370D/E was used in combination with the mutant K360E, the severity of the heterogeneous peaks decreased substantially, resulting in a main peak that eluted close to the times observed at pH5.6 and 6.0, as shown in Figure 5. From these results, CPMv531, and CPMv526 with the addition of K360E were identified as being the most robust variants for standard purification conditions.

All publications, patents, and patent applications discussed and cited herein are hereby incorporated by reference in their entireties. It is understood that the disclosed invention is not limited to the particular methodology, protocols and materials described as these can vary. It is also understood that the terminology used herein is for the purposes of describing particular embodiments only and is not intended to limit the scope of the appended claims.

Those skilled in the art will recognize, or be able to ascertain using no more than routine experimentation, many equivalents to the specific embodiments of the invention described herein. Such equivalents are intended to be encompassed by the following claims.

### EMBODIMENTS

1. An isolated heteromultimer comprising a heterodimeric CH3 domain comprising a first CH3 domain polypeptide and a second CH3 domain polypeptide, the first CH3 domain polypeptide comprising an amino acid modification at position K360, wherein:
   (i) the first CH3 domain polypeptide further comprises an amino acid modification at position K370, and
   (ii) the second CH3 domain polypeptide comprises an amino acid modification at position E357,
   wherein the numbering of amino acid residues is according to the EU index as set forth in Kabat.
2. The isolated heteromultimer according to embodiment 1, wherein the amino acid modification at position K360 is selected from the group consisting of K360E and K360D.
3. The isolated heteromultimer according to any preceding embodiment, wherein the amino acid modification at position K370 is selected from the group consisting of K370E and K370D.
4. The isolated heteromultimer to any preceding embodiment, wherein the amino acid modification at position E357 is selected from the group consisting of E357K, E357H, and E357R.
5. The isolated heteromultimer according to any preceding embodiment, wherein the amino acid modification at position K360 is K360E, the amino acid modification at position K370 is K370D, and the amino acid modification at position E357 is E357K.
6. The isolated heteromultimer according to any preceding embodiment, wherein one CH3 domain polypeptide further comprises an amino acid modification at position K409, and the other CH3 domain polypeptide further comprises an amino acid modification at position D399.
7. The isolated heteromultimer according to embodiment 6, wherein the first CH3 domain polypeptide comprises the amino acid modification at position K409, and the second CH3 domain polypeptide comprises the amino acid modification at position D399.
8. The isolated heteromultimer according to embodiment 6, wherein the first CH3 domain polypeptide comprises the amino acid modification at position D399, and the second CH3 domain polypeptide comprises the amino acid modification at position K409.
9. The isolated heteromultimer according to any one of embodiments 6-8, wherein the amino acid modification at position K409 is selected from the group consisting of K409E and K409D, and wherein the amino acid modification at position D399 is selected from the group consisting of D399K, D399H, and D399R.
10. The isolated heteromultimer according to any one of embodiments 6-9, wherein the amino acid modification at position K409 is K409D, and wherein the amino acid modification at position D399 is D399K.
11. The isolated heteromultimer according to any one of embodiments 6-10, wherein the CH3 domain polypeptide which comprises the amino acid modification at position K409, further comprises an amino acid modification at position K392.
12. The isolated heteromultimer according to embodiment 11, wherein the amino acid modification at position K392 is selected from the group consisting of K392E and K392D.
13. The isolated heteromultimer according to any preceding embodiment, wherein one CH3 domain polypeptide further comprises an amino acid modification at position K439, and the other CH3 domain polypeptide further comprises an amino acid modification at position E356.
14. The isolated heteromultimer according to embodiment 13, wherein the first CH3 domain polypeptide comprises the amino acid modification at position K439, and the second CH3 domain polypeptide comprises the amino acid modification at position E356.
15. The isolated heteromultimer according to embodiment 13, wherein the first CH3 domain polypeptide comprises the amino acid modification at position E356, and the second CH3 domain polypeptide comprises the amino acid modification at position K439.
16. The isolated heteromultimer according to any one of embodiments 13-15, wherein the amino acid modification at position K439 is selected from the group consisting of K439E and K439D, and wherein the amino acid modification at position E356 is selected from the group consisting of E356K, E356H, and E356R.
17. The isolated heteromultimer according to any one of embodiments 13-16, wherein the amino acid modification at position K439 is K439E, and wherein the amino acid modification at position E356 is E356K.
18. The isolated heteromultimer according to any one of embodiments 11-12, wherein the first CH3 domain polypeptide comprises K360E, K370D, K409, and K392D mutations and second CH3 domain polypeptide comprises E357K and D399K mutations.
19. The isolated heteromultimer according to embodiment 18, wherein the first CH3 domain polypeptide further comprises a K439E mutation, and the second CH3 domain polypeptide further comprises a E356K mutation.
20. The isolated heteromultimer according to any preceding embodiment claim, wherein the heterodimeric CH3 domain is comprised by an Fc region based on an IgG Fc region.
21. The isolated heteromultimer according to embodiment 20, wherein the IgG Fc region is an IgG1 Fc region.
22. The isolated heteromultimer according to any preceding embodiment, wherein the heteromultimer is a bispecific or multispecific antibody.
23. A method of stabilizing an isolated heteromultimer at about pH 5.0 wherein the heteromultimer comprises a heterodimeric CH3 domain comprising a first CH3 domain polypeptide and a second CH3 domain polypeptide, wherein:
   (i) the first CH3 domain polypeptide comprises an amino acid modification at position K370, and
   (ii) the second CH3 domain polypeptide comprises an amino acid modification at position E357, the method comprising introducing an amino acid modification at position K360 of the fist CH3 domain, wherein the modification is substitution of K360 for a glutamic or aspartic acid;
   wherein the numbering of amino acid residues is according to the EU index as set forth in Kabat.
24. The method according to embodiment 23, wherein the amino acid modification at position K360 is selected from the group consisting of K360E and K360D.
25. The method according to embodiment 23 or 24, wherein the amino acid modification at position K370 is selected from the group consisting of K370E and K370D.
26. The method according to any one of embodiments 23-25, wherein the amino acid modification at position E357 is selected from the group consisting of E357K, E357H, and E357R.
27. The method according to any one of embodiments 23-25, wherein the amino acid modification at position K360 is K360E, the amino acid modification at position K370 is K370D, and the amino acid modification at position E357 is E357K.
28. The method according to any one of embodiments 23-27, wherein one CH3 domain polypeptide further comprises an amino acid modification at position K409, and the other CH3 domain polypeptide further comprises an amino acid modification at position D399.
29. The method according to embodiment 28, wherein the first CH3 domain polypeptide comprises the amino acid modification at position K409, and the second CH3 domain polypeptide comprises the amino acid modification at position D399.
30. The method according to embodiment 28, wherein the first CH3 domain polypeptide comprises the amino acid modification at position D399, and the second CH3 domain polypeptide comprises the amino acid modification at position K409.
31. The method according to any one of embodiments 28-30, wherein the amino acid modification at position K409 is selected from the group consisting of K409E and K409D, and wherein the amino acid modification at position D399 is selected from the group consisting of D399K, D399H, and D399R.
32. The method according to any one of embodiments 28-31, wherein the amino acid modification at position K409 is K409D, and wherein the amino acid modification at position D399 is D399K.
33. The method according to any one of embodiments 28-32, wherein the CH3 domain polypeptide which comprises the amino acid modification at position K409, further comprises an amino acid modification at position K392.
34. The method according to embodiment 33, wherein the amino acid modification at position K392 is selected from the group consisting of K392E and K392D.
35. The method according to any one of embodiment 23-34, wherein one CH3 domain polypeptide further comprises an amino acid modification at position K439, and the other CH3 domain polypeptide further comprises an amino acid modification at position E356.
36. The isolated heteromultimer according to embodiment 35, wherein the first CH3 domain polypeptide comprises the amino acid modification at position K439, and the second CH3 domain polypeptide comprises the amino acid modification at position E356.
37. The isolated heteromultimer according to embodiment 35, wherein the first CH3 domain polypeptide comprises the amino acid modification at position E356, and the second CH3 domain polypeptide comprises the amino acid modification at position K439.
38. The isolated heteromultimer according to any one of embodiments 35-37, wherein the amino acid modification at position K439 is selected from the group consisting of K439E and K439D, and wherein the amino acid modification at position E356 is selected from the group consisting of E356K, E356H, and E356R.
39. The isolated heteromultimer according to any one of embodiments 35-38, wherein the amino acid modification at position K439 is K439E, and wherein the amino acid modification at position E356 is E356K.
40. The isolated heteromultimer according to any one of embodiments 33-34, wherein the first CH3 domain polypeptide comprises K360E, K370D, K409, and K392D mutations and second CH3 domain polypeptide comprises E357K and D399K mutations.
41. The isolated heteromultimer according to embodiments 40, wherein the first CH3 domain polypeptide further comprises a K439E mutation, and the second CH3 domain polypeptide further comprises a E356K mutation.
42. The isolated heteromultimer according to any one of embodiments 23-41, wherein the heterodimeric CH3 domain is comprised by an Fc region based on an IgG Fc region.
43. The isolated heteromultimer according to embodiment 42, wherein the IgG Fc region is an IgG1 Fc region.
44. The isolated heteromultimer according to any one of embodiments 23-43, wherein the heteromultimer is a bispecific or multispecific antibody.

## Claims

1. An isolated heteromultimer comprising a heterodimeric CH3 domain comprising a first CH3 domain polypeptide and a second CH3 domain polypeptide, the first CH3 domain polypeptide comprising an amino acid modification at position K360, wherein:
(i) the first CH3 domain polypeptide further comprises an amino acid modification at position K370, and
(ii) the second CH3 domain polypeptide comprises an amino acid modification at position E357,
wherein the numbering of amino acid residues is according to the EU index as set forth in Kabat.

2. The isolated heteromultimer according to claim 1, wherein the amino acid modification at position K370 is selected from the group consisting of K370E and K370D.

3. The isolated heteromultimer according to any preceding claim, wherein:
(i) the amino acid modification at position K360 is selected from the group consisting of K360E and K360D;
(ii) the amino acid modification at position E357 is selected from the group consisting of E357K, E357H, and E357R; and/or
(iii) the amino acid modification at position K360 is K360E, the amino acid modification at position K370 is K370E, and the amino acid modification at position E357 is E357K.

4. The isolated heteromultimer according to any preceding claim, wherein one CH3 domain polypeptide further comprises an amino acid modification at position K409, and the other CH3 domain polypeptide further comprises an amino acid modification at position D399, optionally wherein:
(i) the first CH3 domain polypeptide comprises the amino acid modification at position K409, and the second CH3 domain polypeptide comprises the amino acid modification at position D399; or
(ii) the first CH3 domain polypeptide comprises the amino acid modification at position D399, and the second CH3 domain polypeptide comprises the amino acid modification at position K409.

5. The isolated heteromultimer according to claim 4, wherein the amino acid modification at position K409 is selected from the group consisting of K409E and K409D, and wherein the amino acid modification at position D399 is selected from the group consisting of D399K, D399H, and D399R; optionally wherein
the amino acid modification at position K409 is K409D, and wherein the amino acid modification at position D399 is D399K.

6. The isolated heteromultimer according to claim 4 or 5, wherein the CH3 domain polypeptide which comprises the amino acid modification at position K409 further comprises an amino acid modification at position K392, optionally wherein the amino acid modification at position K392 is selected from the group consisting of K392E and K392D.

7. The isolated heteromultimer according to any preceding claim, wherein one CH3 domain polypeptide further comprises an amino acid modification at position K439, and the other CH3 domain polypeptide further comprises an amino acid modification at position E356, optionally wherein:
(i) the first CH3 domain polypeptide comprises the amino acid modification at position K439, and the second CH3 domain polypeptide comprises the amino acid modification at position E356; or
(ii) the first CH3 domain polypeptide comprises the amino acid modification at position E356, and the second CH3 domain polypeptide comprises the amino acid modification at position K439.

8. The isolated heteromultimer according to claim 7, wherein the amino acid modification at position K439 is selected from the group consisting of K439E and K439D, and wherein the amino acid modification at position E356 is selected from the group consisting of E356K, E356H, and E356R, optionally wherein the amino acid modification at position K439 is K439E and wherein the amino acid modification at position E356 is E356K.

9. The isolated heteromultimer according to claim 6, wherein the first CH3 domain polypeptide comprises K360E, K370E, K409, and K392D mutations and the second CH3 domain polypeptide comprises E357K and D399K mutations, optionally wherein the first CH3 domain polypeptide further comprises a K439E mutation, and the second CH3 domain polypeptide further comprises a E356K mutation.

10. The isolated heteromultimer according to any preceding claim, wherein:
(i) the heterodimeric CH3 domain is comprised by an Fc region based on an IgG Fc region, optionally wherein the IgG Fc region is an IgG1 Fc region; and/or
(ii) the heteromultimer is a bispecific or multispecific antibody.

11. A method of stabilizing an isolated heteromultimer at about pH 5.0 wherein the heteromultimer comprises a heterodimeric CH3 domain comprising a first CH3 domain polypeptide and a second CH3 domain polypeptide, wherein:
(i) the first CH3 domain polypeptide comprises an amino acid modification at position K370, and
(ii) the second CH3 domain polypeptide comprises an amino acid modification at position E357, the method comprising introducing an amino acid modification at position K360 of the first CH3 domain, wherein the modification is substitution of K360 for a glutamic or aspartic acid;
wherein the numbering of amino acid residues is according to the EU index as set forth in Kabat.

12. The method according to claim 11, wherein:
(i) the amino acid modification at position K370 is selected from the group consisting of K370E and K370D;
(ii) the amino acid modification at position K360 is selected from the group consisting of K360E and K360D; and/or
(iii) the amino acid modification at position E357 is selected from the group consisting of E357K, E357H, and E357R;
optionally wherein the amino acid modification at position K360 is K360E, the amino acid modification at position K370 is K370E, and the amino acid modification at position E357 is E357K.

13. The method according to claim 11 or 12, wherein one CH3 domain polypeptide further comprises an amino acid modification at position K409, and the other CH3 domain polypeptide further comprises an amino acid modification at position D399, optionally wherein:
(i) the first CH3 domain polypeptide comprises the amino acid modification at position K409, and the second CH3 domain polypeptide comprises the amino acid modification at position D399; or
(ii) the first CH3 domain polypeptide comprises the amino acid modification at position D399, and the second CH3 domain polypeptide comprises the amino acid modification at position K409.

14. The method according to any one of claims 11 to 13, wherein the amino acid modification at position K409 is selected from the group consisting of K409E and K409D and the amino acid modification at position D399 is selected from the group consisting of D399K, D399H, and D399R optionally wherein the amino acid modification at position K409 is K409D and the amino acid modification at position D399 is D399K.

15. The method according to any one of claims 11 to 14, wherein:
(i) the CH3 domain polypeptide which comprises the amino acid modification at position K409 further comprises an amino acid modification at position K392, optionally wherein the amino acid modification at position K392 is selected from the group consisting of K392E and K392D; and/or
(ii) one CH3 domain polypeptide further comprises an amino acid modification at position K439, and the other CH3 domain polypeptide further comprises an amino acid modification at position E356.
